# EUROPEAN PATENT APPLICATION

(11) **EP 4 585 609 A1**
(43) Date of publication of application: **16.07.2025**
(21) Application number: 23887935.7
(22) Date of filing: 06.11.2023
(51) Int. Cl.: C07K 7/64, A61K 51/08, A61P 35/00, A61K 101/02

(54) **RGD DIMER COMPOUND, AND PREPARATION METHOD THEREFOR AND USE THEREOF**

(30) Priority: 07.11.2022 CN 202211381979
(71) Applicant: Yantai Lannacheng Biotechnology Co., Ltd., Yantai Shandong 264199 (CN)
(72) Inventor: CHEN, Xiaoyuan, Yantai, Shandong 264199 (CN); XU, Pengfei, Yantai, Shandong 264199 (CN); WU, Xiaoming, Yantai, Shandong 264199 (CN); GUO, Zhide, Yantai, Shandong 264199 (CN); YANG, Qingbao, Yantai, Shandong 264199 (CN); WEN, Xuejun, Yantai, Shandong 264199 (CN)
(74) Representative: Wang, Bo
(86) International application number: PCT/CN2023/129842
(87) International publication number: WO 2024/099245

(57) **Abstract**

The present invention provides an RGD dimer compound, a preparation method therefor and use thereof, and relates to the fields of nuclear medicine and molecular imaging. The RGD dimer compound has a structure shown in Formula (I) or Formula (I-1). The present invention further provides a radionuclide labeled compound with the RGD dimer compound shown in Formula (I-1) as a ligand, a pharmaceutical composition containing or composed of the RGD dimer compound, and a kit containing or composed of the RGD dimer compound or the pharmaceutical composition. The present invention further provides use of the RGD dimer compound or the pharmaceutical composition in diagnosis or treatment of diseases characterized by over-expression of an integrin αᵥβ₃. The RGD dimer compound and the radionuclide labeled compound of the present invention have higher tumor uptake and a longer retention time and are expected to be used in diagnosis or treatment of diseases characterized by over-expression of the integrin αᵥβ₃.

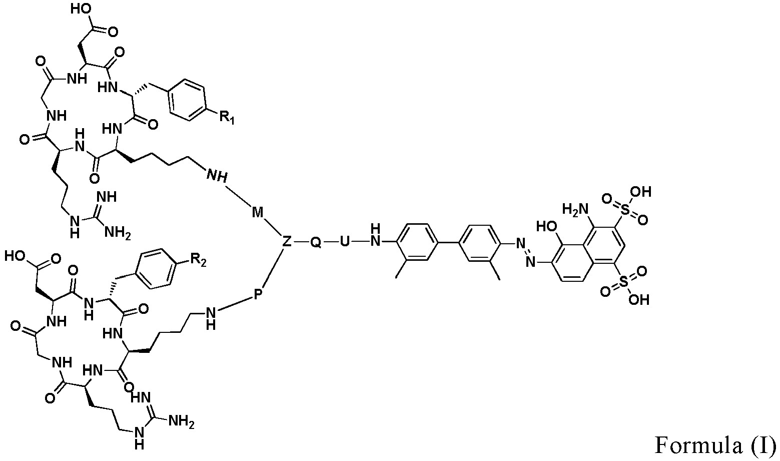

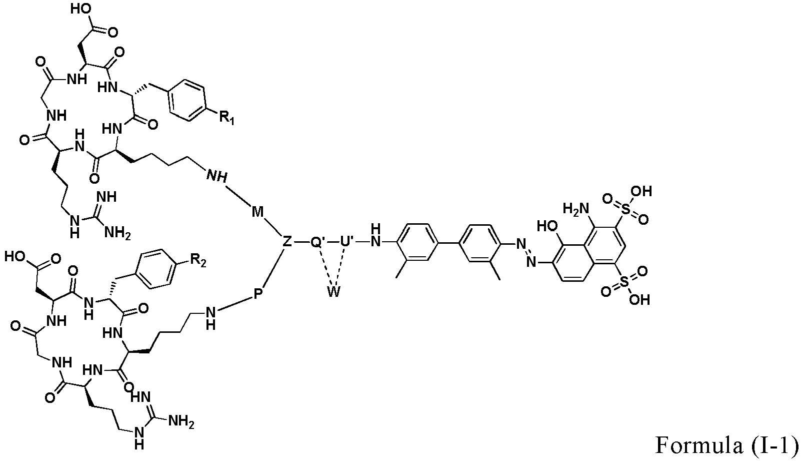

## Description

### TECHNICAL FIELD

The present invention relatesto the fields of nuclear medicine and molecular imaging, and specifically relates to an RGD dimer compound, a preparation method therefor and use thereof.

### BACKGROUND

An integrin αᵥβ₃ is a heterodimeric receptor located on cell surfaces, which is rarely expressed in normal vascular endothelial and epithelial cells, but is expressed at a high level on cell surfaces of a variety of solid tumors, including lung cancer, osteosarcoma, neuroblastoma, breast cancer, prostatic cancer, bladder cancer, glioblastoma, invasive melanoma, etc., and is highly expressed in neovascular endothelial cell membranes of all tumor tissues, indicating that the integrin αᵥβ₃ has an important effect in the processes of growth, invasion and metastasis of tumors. A polypeptide containing an arginine-glycine-aspartic acid (RGD) sequence can specifically bind to the integrin αᵥβ₃. A variety of radionuclide labeled RGD peptides have been successfully used in the research of imaging of a variety of tumor-bearing animal models. In clinical application, ¹⁸F-Galacto-RGD has become a first non-invasive integrin αᵥβ₃ targeted tumor imaging agent in clinical trials, which has been successfully used in PET diagnosis of patients with tumors and shows good biological distribution and specific target recognition in clinical trials of glioblastoma.

However, existing radionuclide labeled RGD cyclic peptides have a short blood half-life and rapid metabolic clearance and cannot be maintained at a therapeutic level at tumor sites. In order to achieve the purpose of therapy, a higher dose or frequent repeated administration is required, which will increase the possibility of occurrence of adverse side effects. Polyethylene glycol modification can reduce a clearance rate of the RGD cyclic peptides, but will lead to immunogenicity and reduce the bioavailability.

In conclusion, the radionuclide labeled RGD cyclic peptides in the prior art have the limitations of low tumor uptake and a short retention time, making it unable to achieve the purpose of therapy. In addition, a high dose and highly frequent administration to achieve the purpose of therapy also have the risk of increasing adverse side effects, making it difficult to realize wide application in clinical practice.

### SUMMARY

Based on the above background, in order to solve the problems that radionuclide labeled RGD peptides have low tumor uptake and a short retention time, a primary purpose of the present invention is to develop a connector of truncated Evans Blue (tEB) and an integrin αᵥβ₃ specific ligand RGD dimer peptide (2RGD). The connector is characterized in that it can effectively bind to serum albumin through a truncated Evans Blue structure to use the albumin as a delivery vector of the RGD dimer peptide, thereby prolonging a half-life of the RGD dimer peptide in peripheral blood, increasing uptake and enrichment of the RGD dimer peptide in tumors, and prolonging a retention time.

Another purpose of the present invention is to research and develop a radionuclide labeled compound based on a structure of the connector.

Another purpose of the present invention is to provide a method for preparing the connector and a method for preparing the radionuclide labeled compound.

Another purpose of the present invention is to provide use of the connector or the radionuclide labeled compound in diagnosis or treatment of diseases characterized by over-expression of an integrin αᵥβ₃.

The above purposes of the present invention are achieved through the following technical solutions.

In a first aspect, the present invention provides an RGD dimer compound, which has a truncated Evans Blue modified RGD dimer structure. The structure of the compound is shown in Formula (I) or (I-1) below, 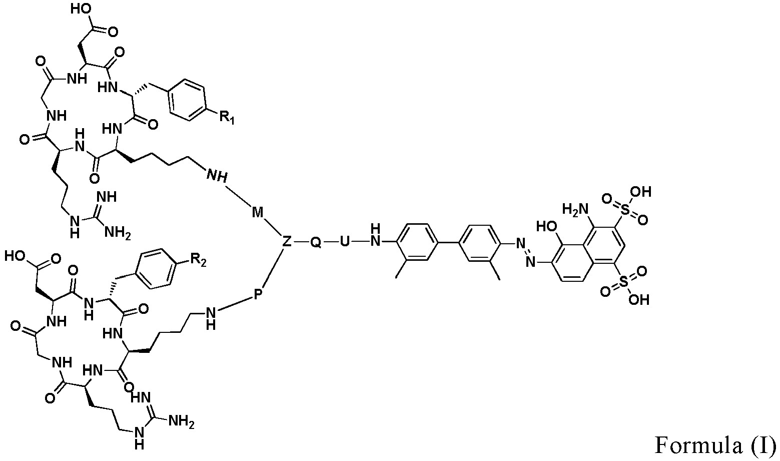 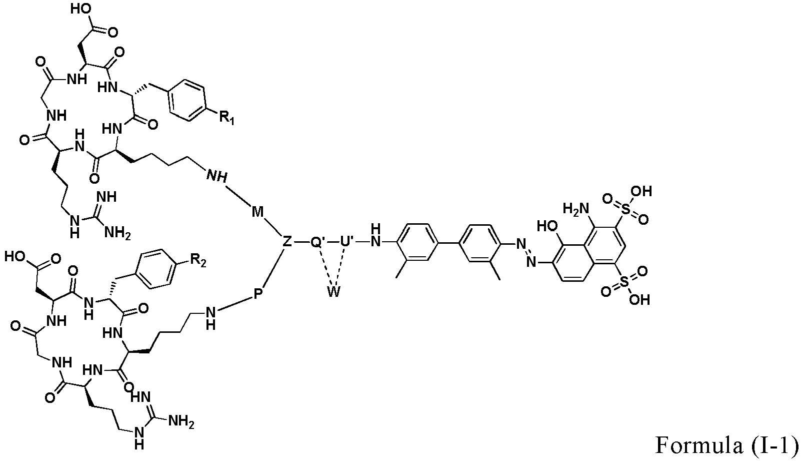 where:
R₁ and R₂ are independently selected from OH or H;
M and P are the same or different, and are independently selected from 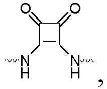 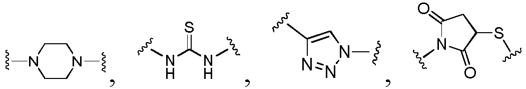 or -(CH₂)ₙ-; when the M and the P are -(CH₂)ₙ-, n is an integer ranging from 0 to 30, each -CH₂- is individually substituted or unsubstituted with -O-, -NH-, -(CO)-, -NH(CO)-, or -(CO)-NH-, and substitution occurs when no two adjacent -CH₂- groups are substituted;
Z is selected from 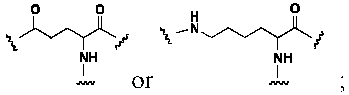
Q and U exist or do not exist, and are independently selected from 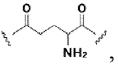 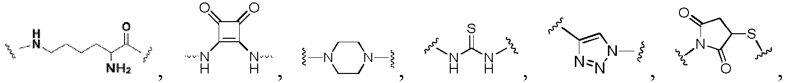 or -(CH₂)ₙ-; when the Q and the U are -(CH₂)ₙ-, n is an integer ranging from 0 to 30, each -CH₂- is individually substituted or unsubstituted with -O-, -NH-, -(CO)-, -NH(CO)-, or -(CO)-NH-, and substitution occurs when no two adjacent -CH₂- groups are substituted;
Q' and U' exist or do not exist, and any one structure in the Q' or the U' is connected with W; when the Q' or the U' exists and is connected with the W, the Q' and the U' can be independently selected from 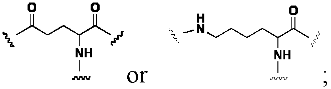 when the Q' or the U' exists and is not connected with the W, the Q' and the U' can be independently selected from 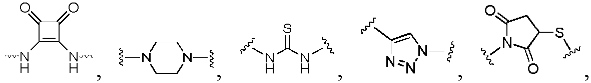 or -(CH₂)ₙ-; when the Q' and the U' are -(CH₂)ₙ-, n is an integer ranging from 0 to 30, each -CH₂- is individually substituted or unsubstituted with -O-, -NH-, -(CO)-, -NH(CO)-, or -(CO)-NH-, and substitution occurs when no two adjacent -CH₂- groups are substituted; and
the W is a group capable of being chelated with a radionuclide, preferably any one structure selected from 1,4,7,10-tetraazacyclododecane-N,N',N,N'-tetraacetic acid (DOTA), ethylenediamine tetraacetic acid (EDTA), 1,4,7-triazacyclononane-1,4,7-triacetic acid (NOTA), triethylenetetramine (TETA), iminodiacetic acid, diethylenetriamine-N,N,N',N',N'-pentaacetic acid (DTPA), bis-(carboxymethylimidazole)glycinate, or 6-hydrazinonicotinic acid (HYNIC).

In a preferred solution of the present invention, the compound having the structure shown in Formula (I-1) in the first aspect can be any one shown in Formula (II-1) to Formula (II-16) below:

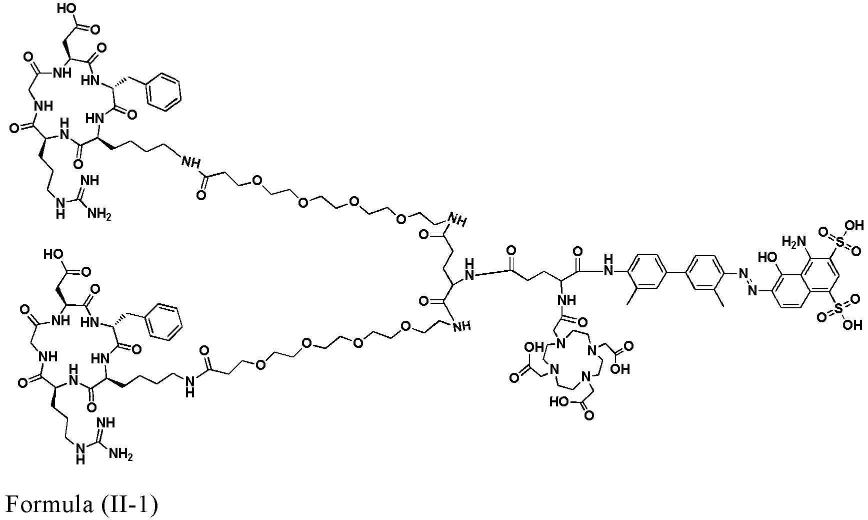

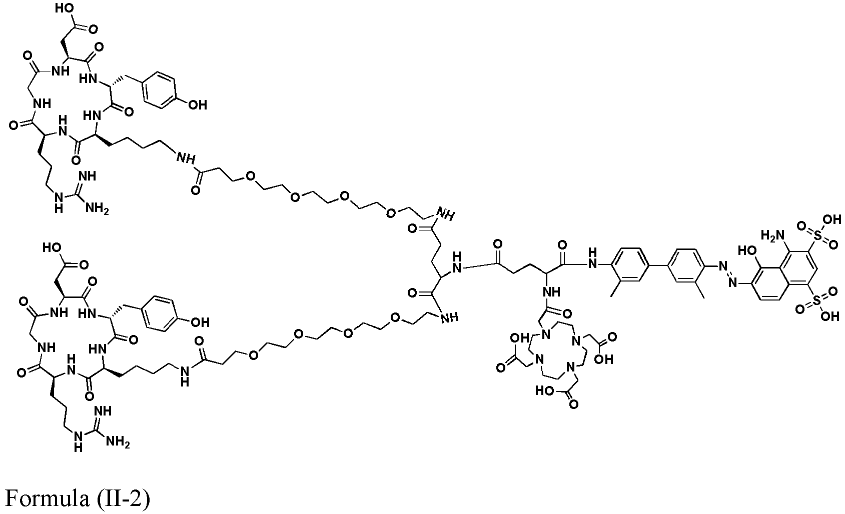

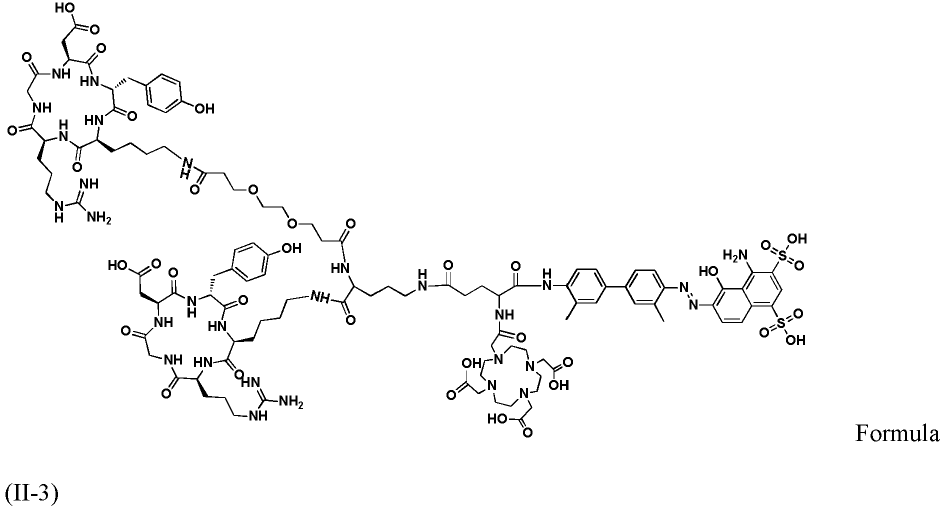

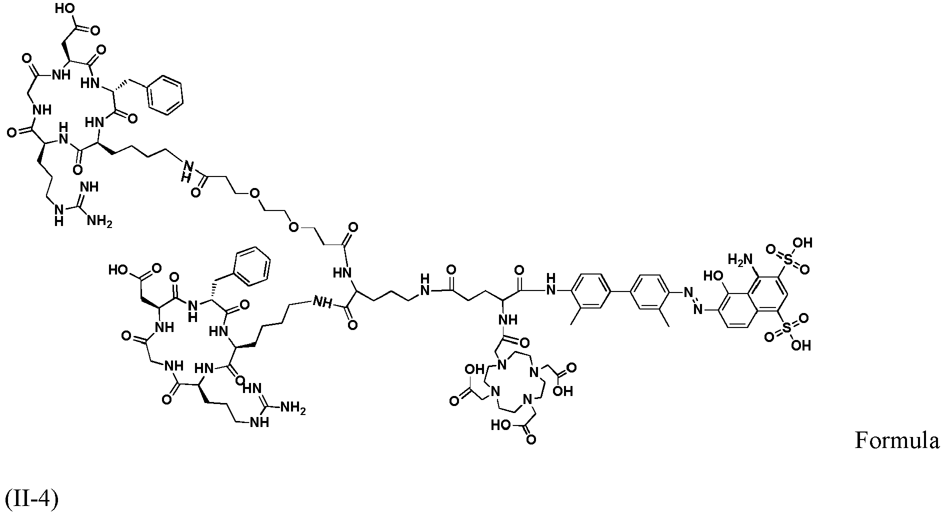

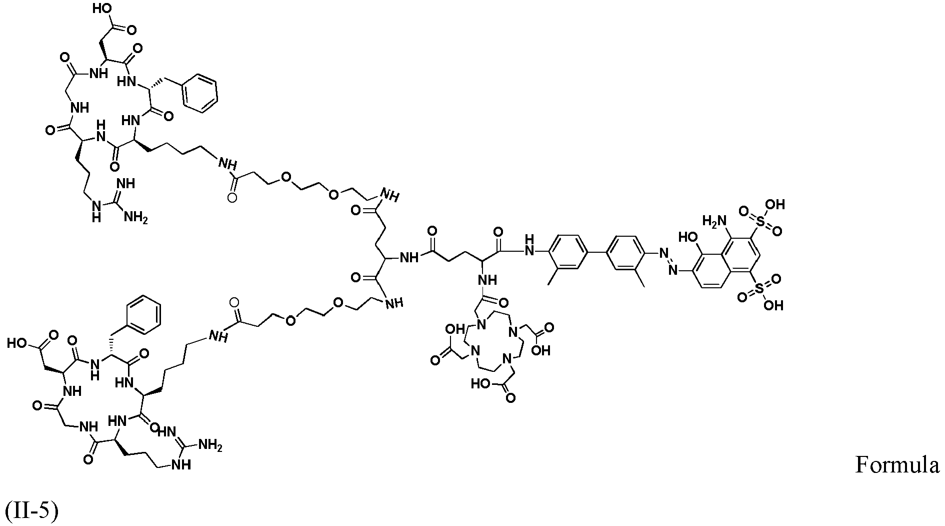

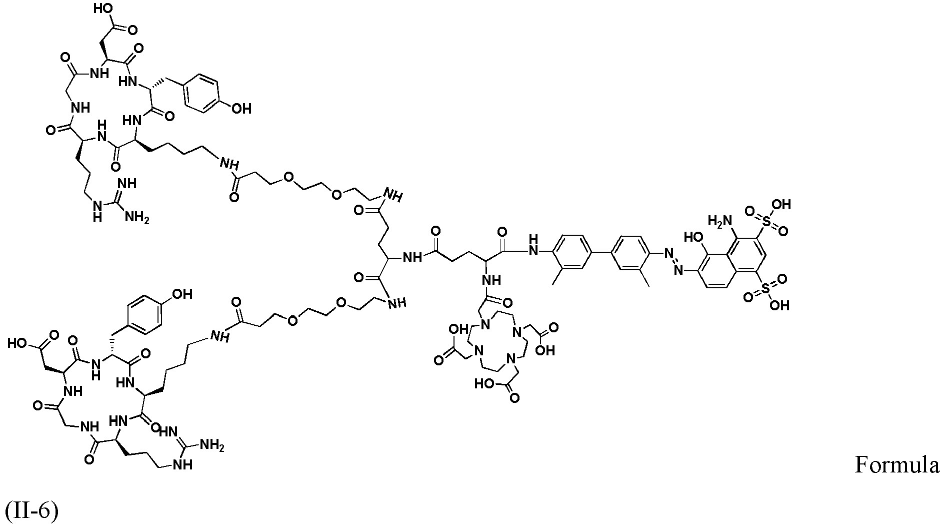

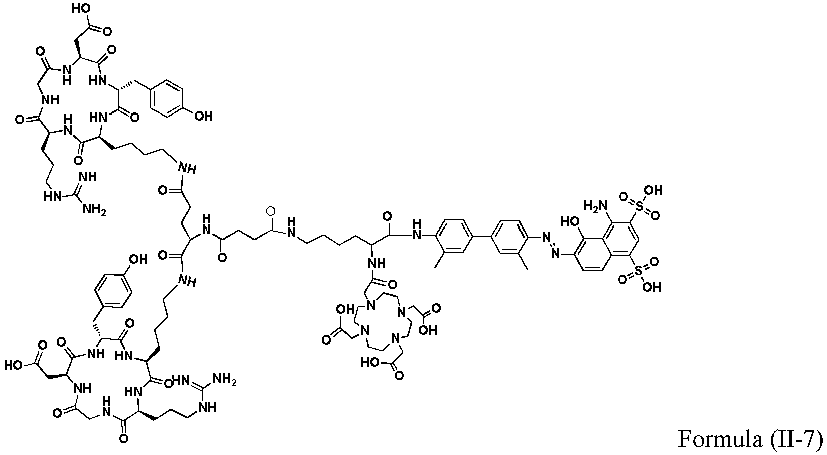

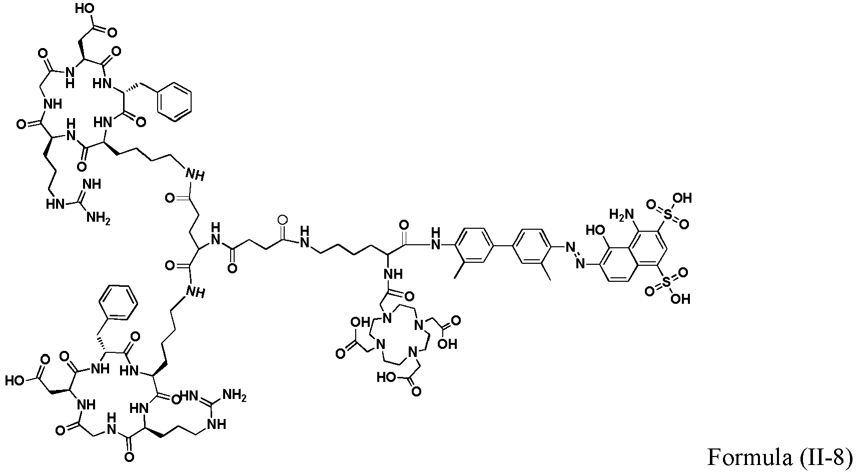

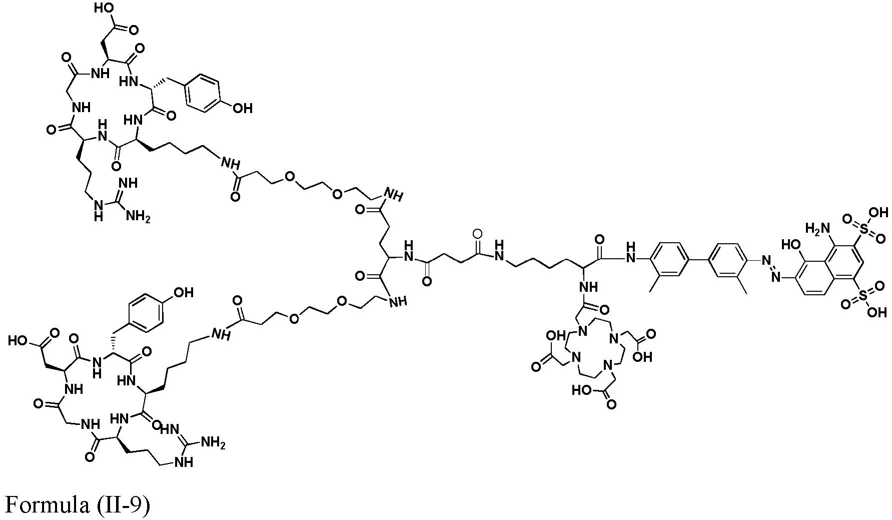

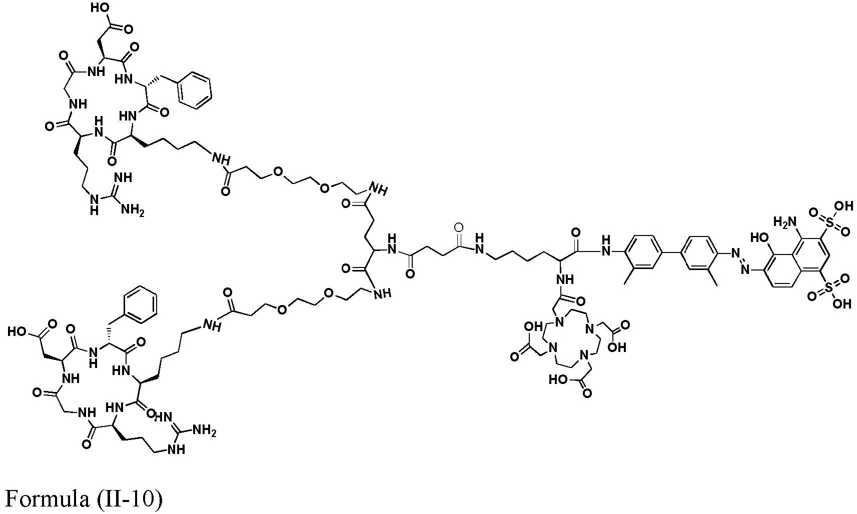

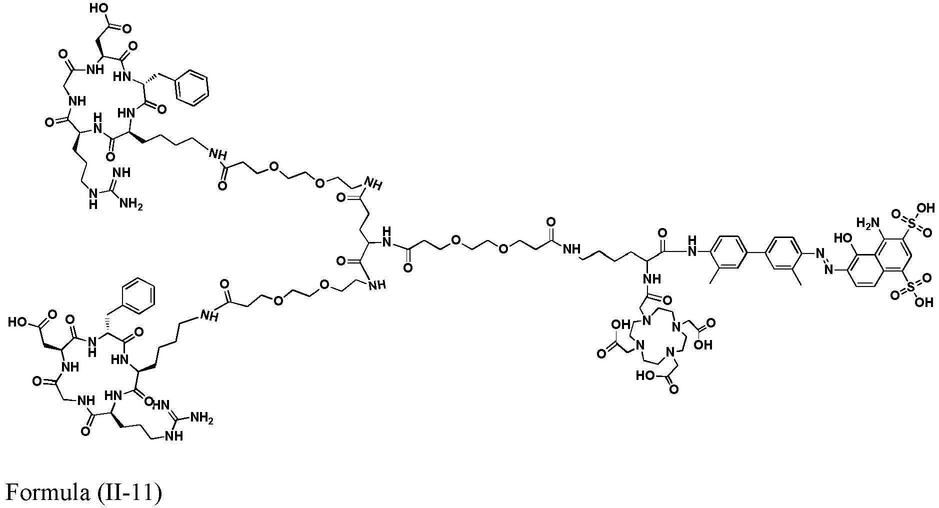

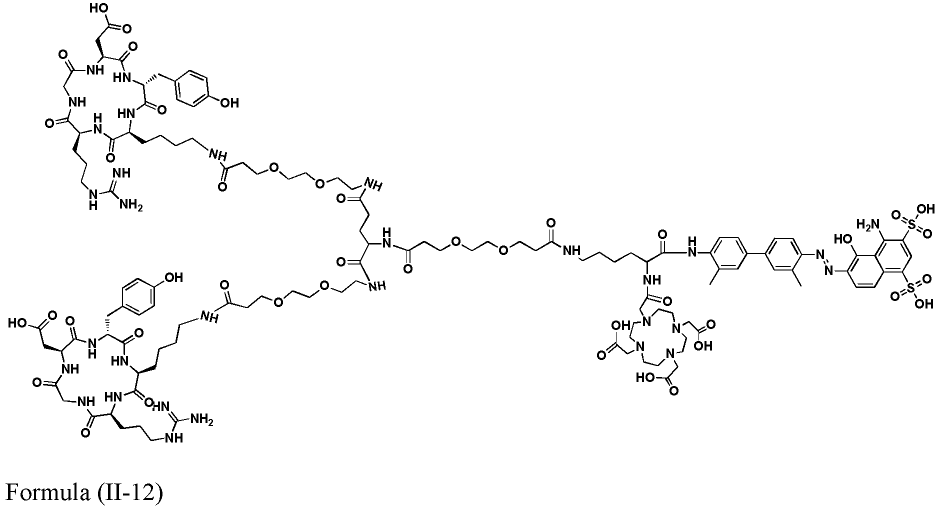

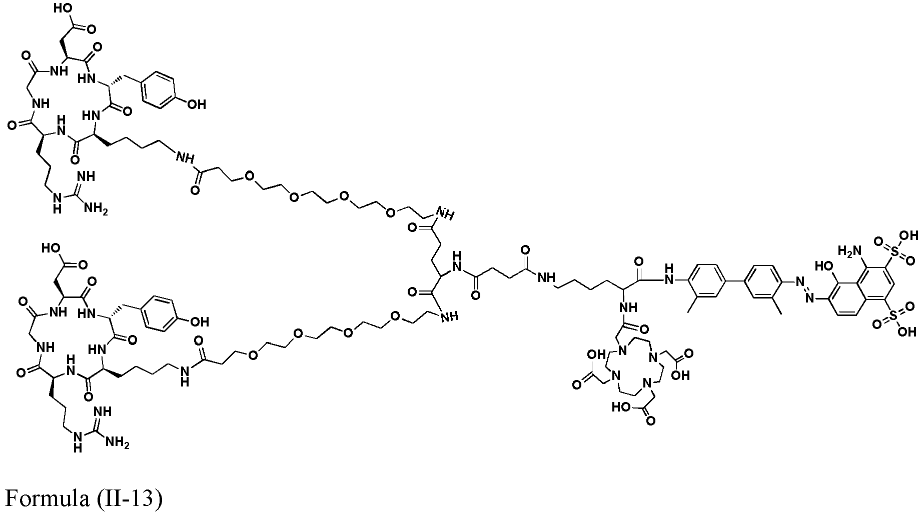

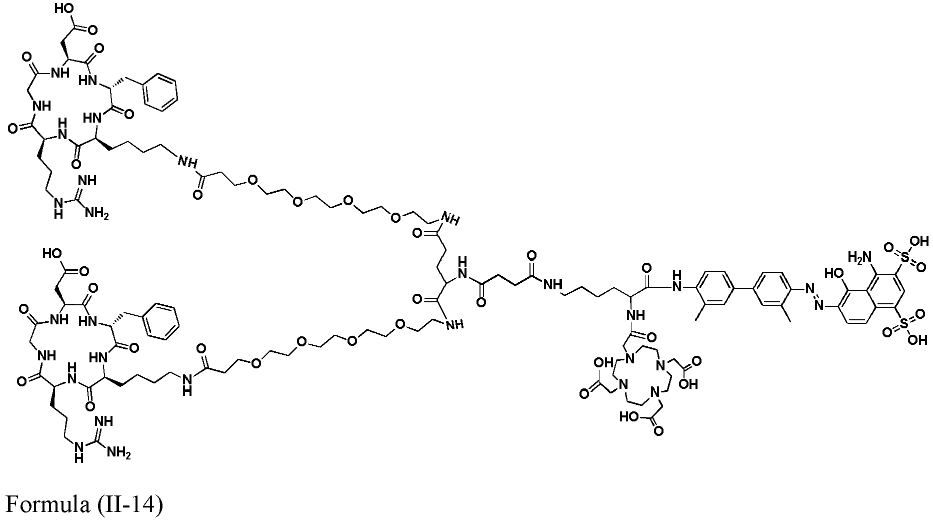

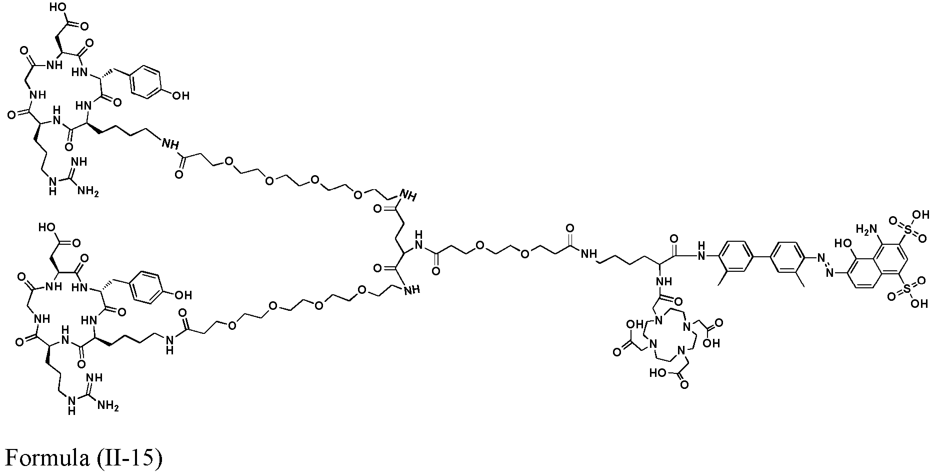

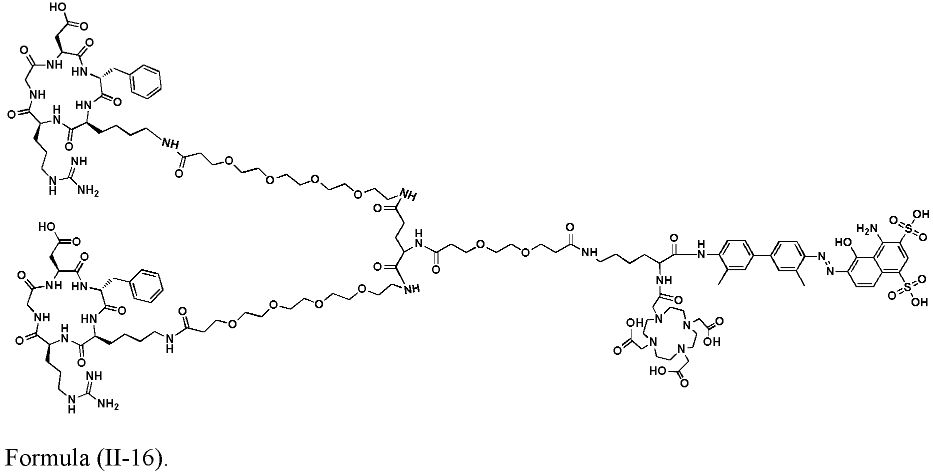

In the first aspect, the present invention further provides a radionuclide labeled compound, which is formed by chelating a radioisotope on a group W capable of being chelated with a radionuclide with any one compound shown in Formula (I-1) as a ligand. The radioisotope is preferably an isotope emitting α rays, an isotope emitting β rays, an isotope emitting γ rays, an isotope emitting Auger electrons, or an isotope emitting X rays, etc.; the radioisotope is further preferably any one of ¹⁸F, ⁵¹Cr, ⁶⁴Cu, ⁶⁷Cu, ⁶⁷Ga, ⁶⁸Ga, ⁸⁹Zr, ¹¹¹In, ^{99m}Tc, ¹⁸⁶Re, ¹⁸⁸Re, ¹³⁹La, ¹⁴⁰La, ¹⁷⁵Yb, ¹⁵³Sm , ¹⁶⁶Ho, ⁸⁶Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg , ²¹¹At, ¹⁵¹Eu, ¹⁵³Eu, ¹⁶⁹Eu, ²⁰¹Tl, ²⁰³Pb, ²¹²Pb, ¹⁹⁸Au, ²²⁵Ac, ²²⁷Th, or ¹⁹⁹Ag; and the radioisotope is more preferably ¹⁸F, ⁶⁴Cu, ⁶⁸Ga, ⁸⁹Zr, ⁹⁰Y, ¹¹¹In, ^{99m}Tc, ¹⁷⁷Lu, ¹⁸⁸Re, or ²²⁵Ac.

The present invention further provides pharmaceutically acceptable tautomers, racemates, hydrates, solvates or salts of all of the compounds in the first aspect.

In a second aspect, the present invention first provides a method for preparing the RGD dimer compound shown in Formula (I) in the first aspect, which includes the following steps:
(1) subjecting truncated Evans blue to an amide condensation reaction with carboxyl of glutamic acid, lysine, or cysteine with amino protection to obtain an intermediate compound A with amino protection;
(2) subjecting c(RGDfK) or c(RGDyK) to a reaction with tert-butoxycarbonyl-tetrapolyethylene glycol-succinimide acrylate to remove tert-butoxycarbonyl (Boc) protection, followed by a reaction with glutamic acid diactivated ester with fluorenylmethyloxycarbonyl (Fmoc) protection to prepare an RGD dimer peptide; and
(3) subjecting the intermediate compound A obtained in step (1) to an amide condensation reaction with the RGD dimer peptide obtained in step (2), and then removing the Boc protection by p-methylbenzene sulfonic acid to obtain the RGD dimer compound shown in Formula (I).

The present invention further provides a method for preparing the RGD dimer compound shown in Formula (I-1) in the first aspect, which includes: connecting amino of the RGD dimer compound shown in Formula (I) obtained in step (3) above to a group capable of being chelated with a radionuclide to obtain the RGD dimer compound shown in Formula (I-1).

Furthermore, the present invention further provides a method for preparing the radionuclide labeled compound in the first aspect, which includes: labeling the RGD dimer compound shown in Formula (I-1) with a radionuclide by a conventional wet method or freeze-drying method to obtain the radionuclide labeled compound of the present invention.

In a third aspect, the present invention provides a pharmaceutical composition, which includes or is composed of i) any one RGD dimer compound or the radionuclide labeled compound in the first aspect, and ii) at least one pharmaceutically acceptable carrier and/or excipient.

In a fourth aspect, the present invention provides use of the RGD dimer compound in the first aspect, the radionuclide labeled compound in the first aspect, or the pharmaceutical composition in the third aspect in preparation of drugs for diagnosis or treatment of diseases characterized by over-expression of an integrin αᵥβ₃ in animals or human objects.

The diseases characterized by over-expression of the integrin αᵥβ₃ are preferably selected from lung cancer, glioma, neuroglioma, breast cancer, pancreatic cancer, small intestine cancer, colon cancer, rectal cancer, head and neck cancer, ovarian cancer, hepatocellular carcinoma, esophageal cancer, hypopharyngeal carcinoma, nasopharyngeal carcinoma, laryngeal carcinoma, myeloma cells, bladder cancer, cholangiocellular carcinoma, clear cell renal cell carcinoma, neuroendocrine neoplasm, carcinogenic osteomalacia, sarcoma, cancer of unknown primary (CUP), thymic carcinoma, astrocytoma, cervical cancer, or prostatic cancer.

In a fifth aspect, the present invention provides a kit, which includes or is composed of any one RGD dimer compound in the first aspect of the present invention, the radionuclide labeled compound, or the pharmaceutical composition in the third aspect.

Compared with the prior art, the present invention has the following beneficial effects. The RGD dimer compound having the structure can improve the tumor uptake and the retention time and is expected to be used in diagnosis or treatment of diseases characterized by over-expression of the integrin αᵥβ₃.

### BRIEF DESCRIPTION OF DRAWINGS

FIG. 1 is a mass spectrogram of a compound H in Example 1 of the present invention.
FIG. 2 is a diagram showing ⁶⁸Ga labeled HPLC quality control results of the compound H in Example 1 of the present invention.
FIG. 3 shows ⁶⁸Ga labeled in vitro stability analysis of the compound H in Example 1 of the present invention.
FIG. 4 shows MicroPET imaging results of a ⁶⁸Ga labeled complex of the compound H in Example 1 of the present invention in U87 tumor-bearing mice.

### DETAILED DESCRIPTION OF EMBODIMENTS

The technical solutions of the present invention are further explained and described below in conjunction with specific embodiments and attached drawings.

### Example 1: Preparation of 2RGD-EB (compound (II-1))

### Preparation of a compound c(RGDfK)-PEG₄:

Fmoc-PEG₄-CH₂CH₂COOH (compound i) (1.46 g, 3.0 mmol) was dissolved in DMF, and then DCC (0.68 g, 3.3 mmol) and HOSu (0.38 g, 3.3 mmol) were added to carry out a reaction at room temperature for 6 h. Filtration was carried out, TEA (0.90 g, 9.0 mmol) was added to a filtrate, and then c(RGDfK) (compound ii) (2.23 g, 3.6 mmol) was added to carry out a reaction at room temperature for 3 h. A reaction solution was spin-dried, dissolved in 25% DEA/THF to carry out a reaction at room temperature for 4 h, concentrated until a small amount of the solution was remained, and then added to 10 times the volume of ethyl ether until a large amount of a solid was precipitated. Then filtration was carried out to obtain crude c(RGDfK)-PEG₄, and the crude product was purified by reversed-phase preparative liquid chromatography to obtain fine c(RGDfK)-PEG₄, where an eluent (including a solution A: ultrapure water containing 1/1000 volume of trifluoroacetic acid; and a solution B: acetonitrile) was used.

### Synthesis of a compound 2(RGDfK)PEG₄-Glu:

Boc-Glu-OH (0.4 g, 2.0 mmol) was dissolved in DMF, and then DCC (0.45 g, 2.2 mmol) and HOSu (0.25 g, 2.2 mmol) were added to carry out a reaction at room temperature for 6 h. Filtration was carried out, TEA (0.60 g, 6.0 mmol) was added to a filtrate, and then the c(RGDfK)-PEG₄ (2.61 g, 2.4 mmol) was added to carry out a reaction at room temperature for 3 h. A reaction solution was spin-dried, dissolved in TFA to carry out a reaction at room temperature for 10 min, and then added to 10 times the volume of ethyl ether until a large amount of a solid was precipitated. Then filtration was carried out to obtain crude 2(RGDfK)PEG₄-Glu, and the crude product was purified by reversed-phase preparative liquid chromatography to obtain fine 2(RGDfK)PEG₄-Glu, where an eluent (including a solution A: ultrapure water containing 1/1000 volume of trifluoroacetic acid; and a solution B: acetonitrile) was used. Then, the pH of the fine 2(RGDfK)PEG₄-Glu was adjusted to neutral with TEA, the reversed-phase preparative liquid chromatography was carried out again, and freeze-drying was carried out to obtain finished 2(RGDfK)PEG₄-Glu, where an eluent (including a solution A: ultrapure water; and a solution B: acetonitrile) was used.

### Preparation of a compound A:

Under the condition of room temperature, o-tolidine (50.00 g, 235.53 mmol) was added to 450 ml of dichloromethane and stirred for dissolution, and a dichloromethane solution (50 ml) of di-tert-butyl dicarbonate (51.40 g, 235.53 mmol) was added dropwise to carry out a reaction at room temperature for 42 h. Filtration was carried out, a filtrate was washed with a 0.1 mol/L hydrochloric acid solution for 3 times with 500 ml each time, an organic phase was washed with 500 ml of water and then dried with anhydrous sodium sulfate, and then the organic phase was subjected to rotary evaporation under reduced pressure. After spin-drying was carried out, re-dissolution was carried out with 500 ml of ethyl acetate, 60 ml of a 4 mol/L HCl/EA solution was added, then 1 L of methyl tert-butyl ether was added, cooling was carried out to 0-10°C for crystallization, filtration was carried out, and drying was carried out at 45°C to obtain a compound A (47.32 g, yield: 64.31%)

### Preparation of a compound B:

Under the condition of room temperature, the compound A (50.00 g, 143.42 mmol), DIPEA (46.37 g, 358.55 mmol), Fmoc-Glu(OtBu)-OH (67.12 g, 157.77 mmol) and HATU (60.00 g, 157.77 mmol) were sequentially added to 400 ml of acetonitrile and dissolved to carry out a reaction at room temperature for 18 h. After the reaction was completed, a reaction solution was spin-dried under reduced pressure, redissolved in 500 ml of dichloromethane, and then sequentially washed with 500 ml of a saturated sodium bicarbonate solution and 500 ml of pure water for 2 times. An organic phase was spin-dried under reduced pressure and purified by silica gel column chromatography (dichloromethane:ethyl acetate=20:1). An eluent product was collected, spin-dried under reduced pressure, beaten with 1 L of methyl tert-butyl ether, filtered and then dried at 45°C to obtain a compound B (103.49 g, yield: 89.82%).

### Preparation of a compound C:

Under the condition of room temperature, the compound B (56.00 g, 77.84 mmol) was added to 560 ml of dichloromethane and stirred for dissolution, and then 112 ml of trifluoroacetic acid was added and stirred to carry out a reaction at 30°C for 2 h. After the reaction was completed, a reaction solution was added to 2.24 L of methyl tert-butyl ether to precipitate a solid, filtered and then dried at 45°C to obtain a compound C (49.12 g, yield: 97.46%).

### Preparation of a compound D:

Under the condition of room temperature, the compound C (27.64 g, 49.04 mmol) was added to a mixed solution of 750 ml of acetonitrile and 200 ml of purified water, stirred for dissolution and cooled to -5°C to 0°C in an ice bath. 81.6 ml of 2 mol/L hydrochloric acid was added, and then a sodium nitrite aqueous solution (3.38 g, 49.04 mmol, 50 ml of water) was added and stirred to carry out a reaction for 30 min. A resulting diazonium salt solution was slowly added dropwise to an aqueous solution (200ml) of 1-amino-8-naphthol-2,4-disulfonate monosodium salt (16.74 g, 49.04 mmol) and sodium bicarbonate (24.72 g, 294.24 mmol), where the temperature was controlled at 0-5°C in the dropping process. After the dropping was completed, heat preservation was conducted to carry out a reaction at 0-5°C for 2 h, and a reaction solution was spin-dried under reduced pressure and purified by preparative liquid chromatography to obtain a compound D (19.91 g, yield: 45.28%).

### Preparation of a compound F:

Under the condition of room temperature, the compound D (0.5576 g, 0.624 mmol) and HATU (0.2419 g, 0.636 mmol) were added to 50 ml of DMF and stirred at room temperature for 30 min. The 2(RGDfK)PEG₄-Glu (1.1874 g, 0.655 mmol) was added and stirred to carry out a reaction at room temperature for 4 h. After the reaction was completed, 11 ml of piperidine was added to a reaction flask and continuously stirred to carry out a reaction for 4.5 h. After the reaction was completed, spin-drying under reduced pressure was carried out, and purification by preparative liquid chromatography was carried out to obtain a compound F (0.80 g, two-step yield: 51.97%).

### Preparation of a compound H:

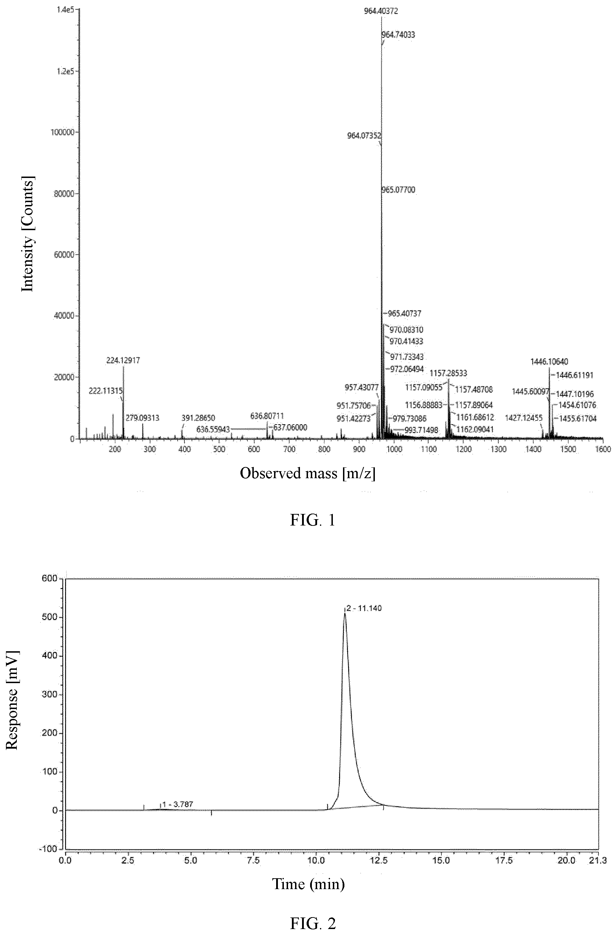

Under the condition of room temperature, the compound F (0.3662 g, 0.148 mmol), DIPEA (0.2304 g, 1.78 mmol) and DOTA-TRIS-TBU-ESTERNHS (0.2982 g, 0.42 mmol) were added to 7.3 ml of DMF, and heat preservation was conducted to carry out a reaction at 30°C for 40 h. After the reaction was completed, a reaction solution was spin-dried under reduced pressure to obtain 0.7332 g of a crude compound G. The crude compound G was added to 7 ml of trifluoroacetic acid and stirred for dissolution to carry out a reaction 30°C for 3 h. After the reaction was completed, a reaction solution was added to 40 ml of methyl tert-butyl ether, suction filtration was carried out, and a solid was dried under reduced pressure to obtain 0.5494 g of a crude compound H. The crude compound H was purified by preparative liquid chromatography to obtain a compound H (0.1426 g, two-step yield: 31.89%). FIG. 1 is a mass spectrogram of the compound H. [M+K+H+H]³⁺/3=964.

A synthesis route of the above steps is as follows:

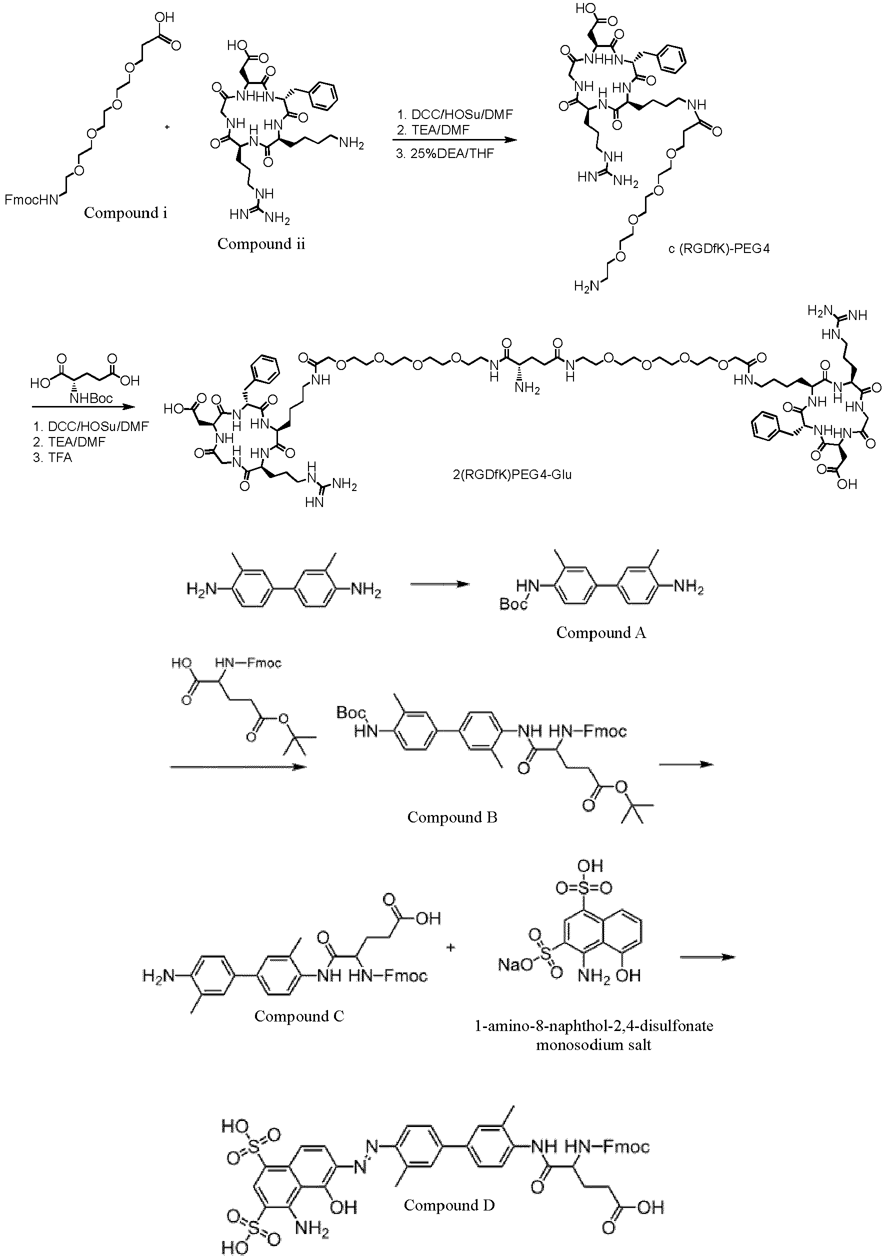

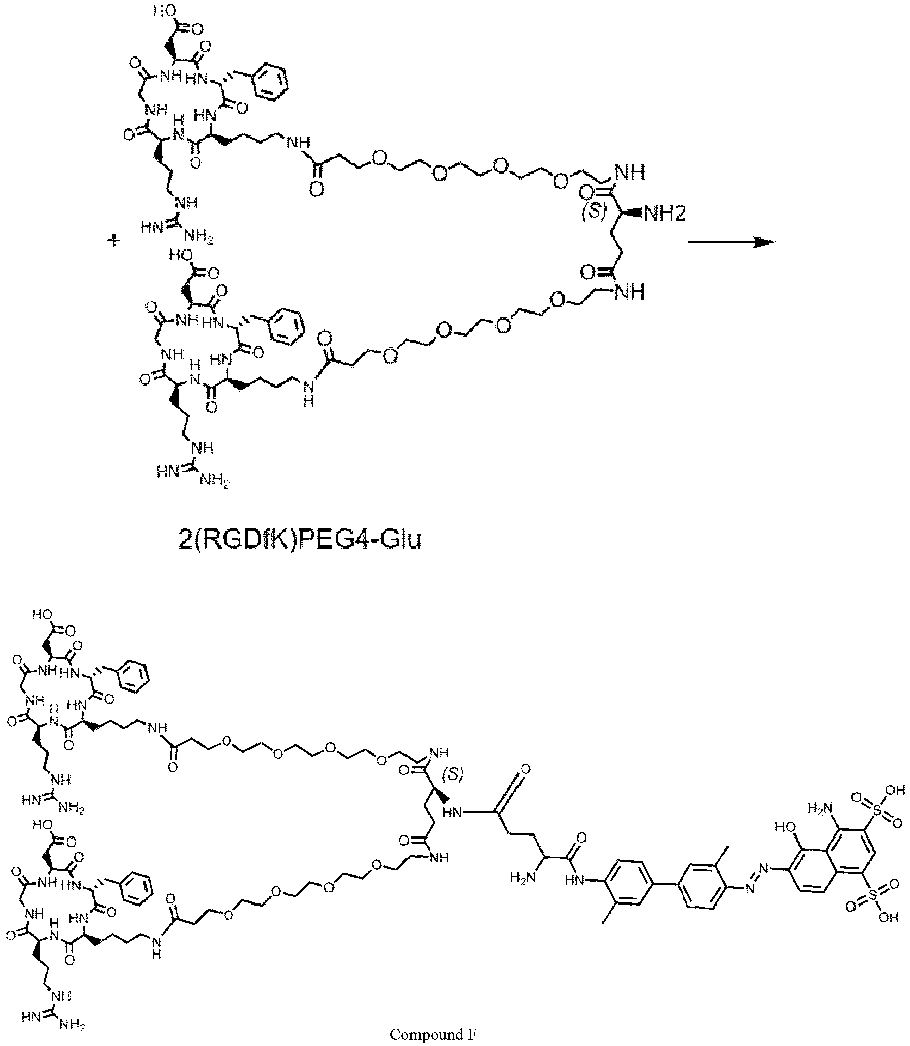

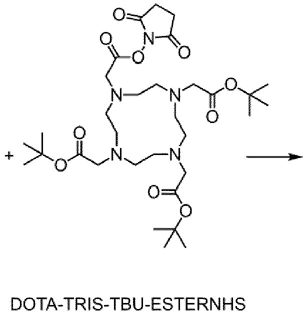

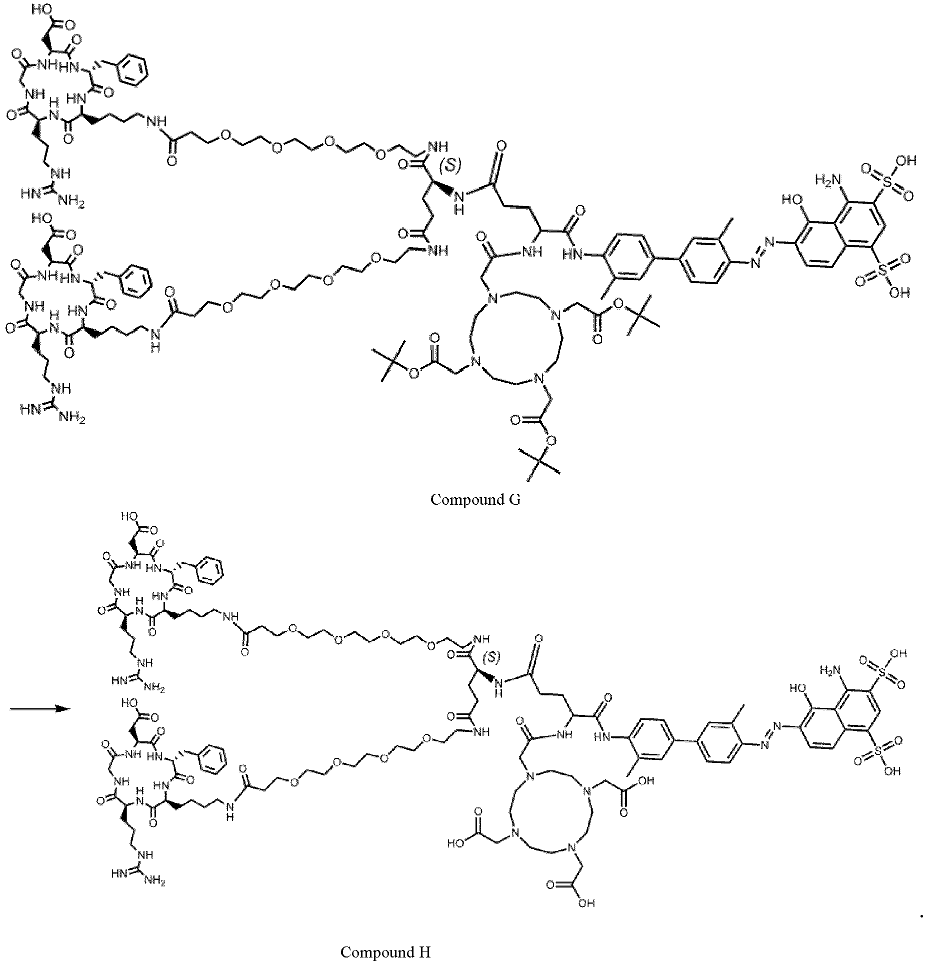

### Examples 2-16

Structures of compounds in Examples 2-16 are shown in Formula (II-2) to Formula (II-16), respectively. Preparation methods for the compounds can include replacing some raw materials based on the preparation in Example 1, for example, replacing the c(RGDfK) with c(RGDyK), replacing N-[(9H-fluoren-9-ylmethoxy)carbonyl]-L-glutamic acid with N-Boc-N'-Fmoc-L-lysine, replacing tert-butoxycarbonyl-tetrapolyethylene glycol-succinimide acrylate with bis(succinimide)-4,7-dioxodecanediate, etc., to obtain the following corresponding structures:

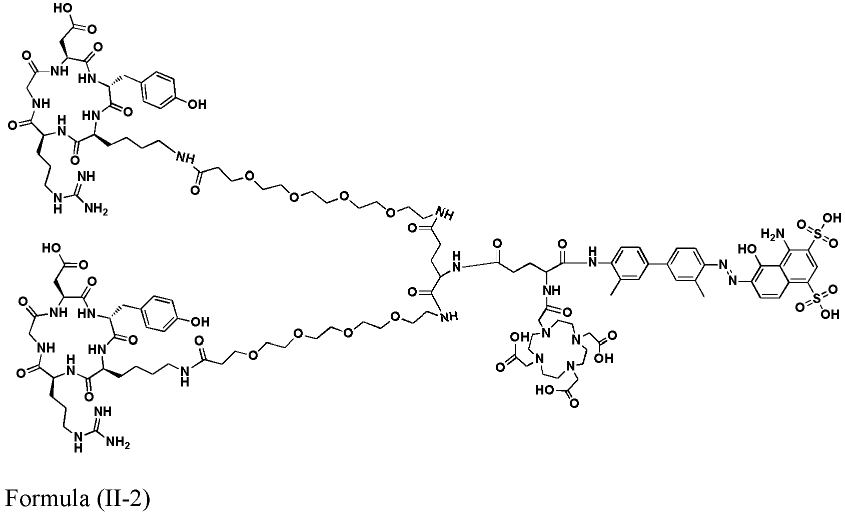

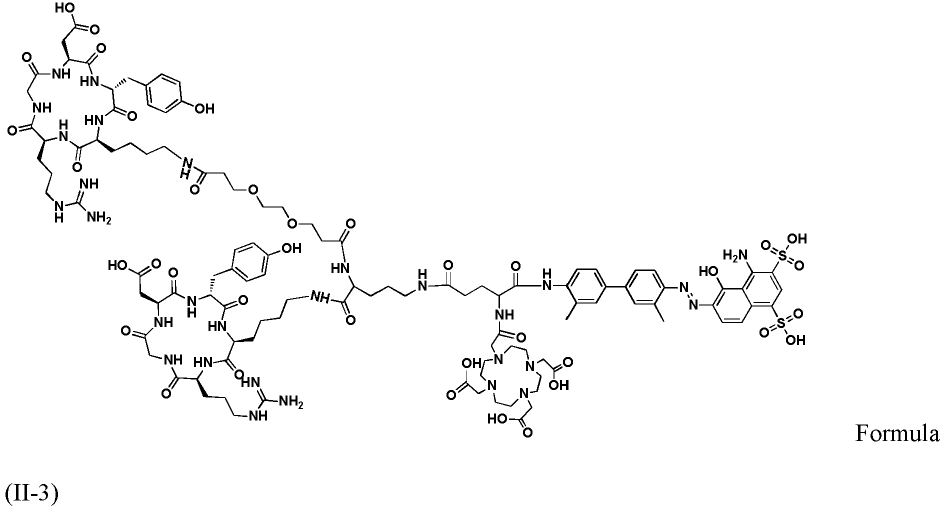

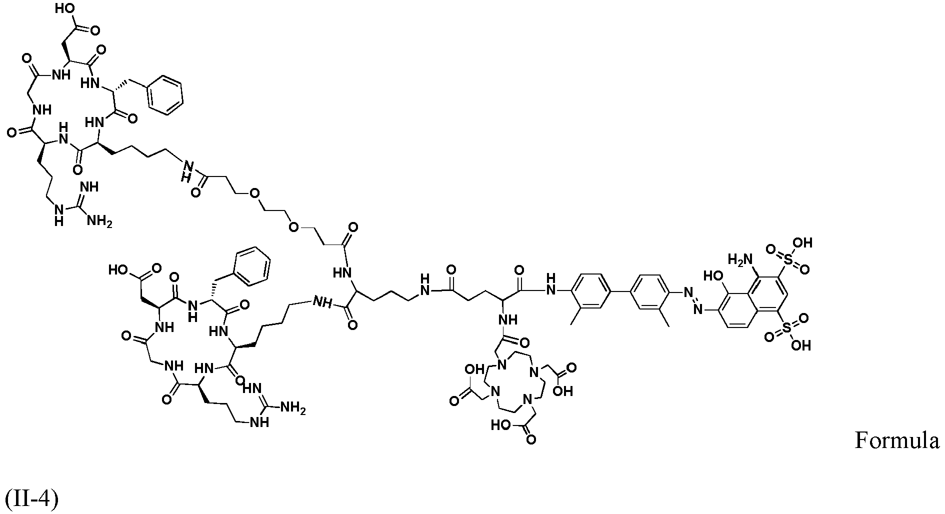

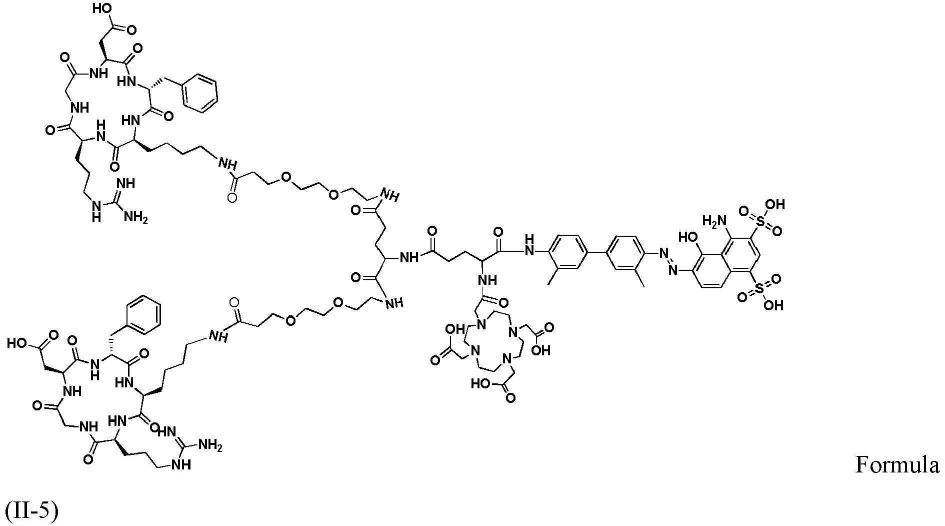

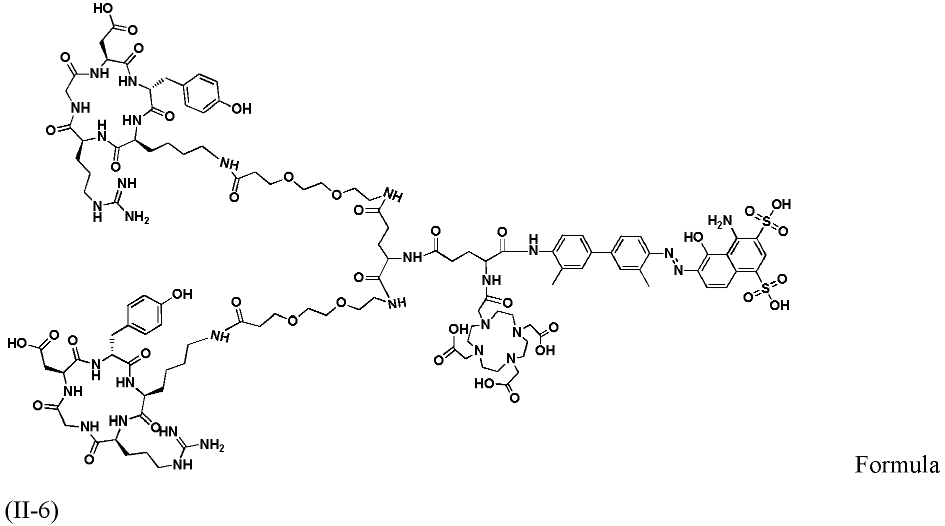

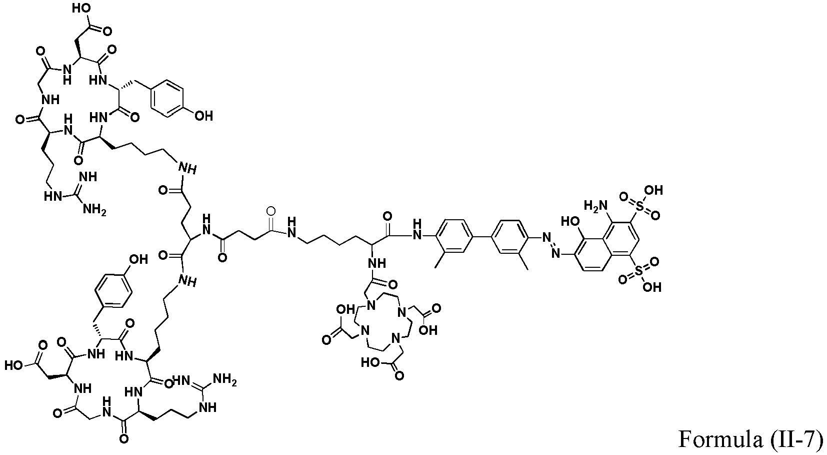

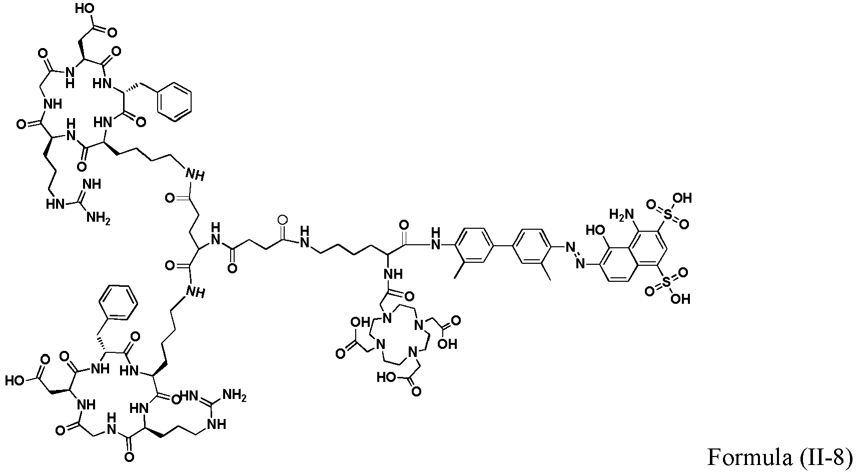

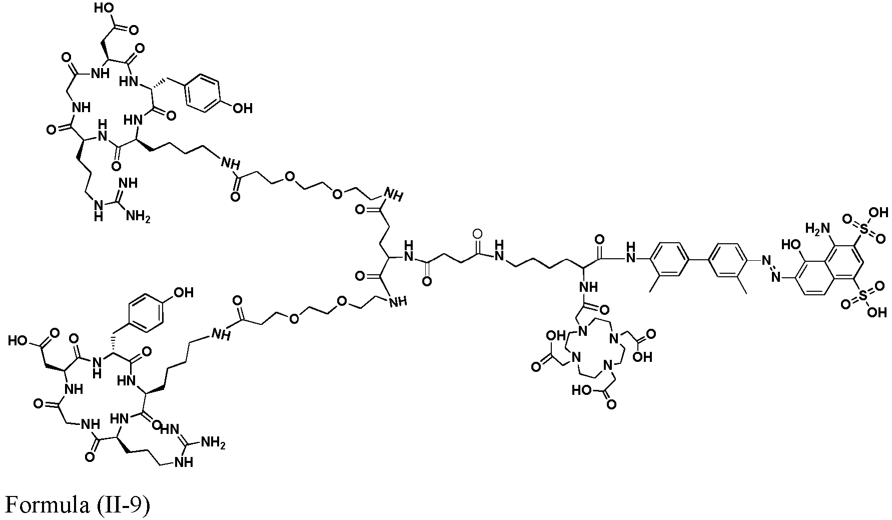

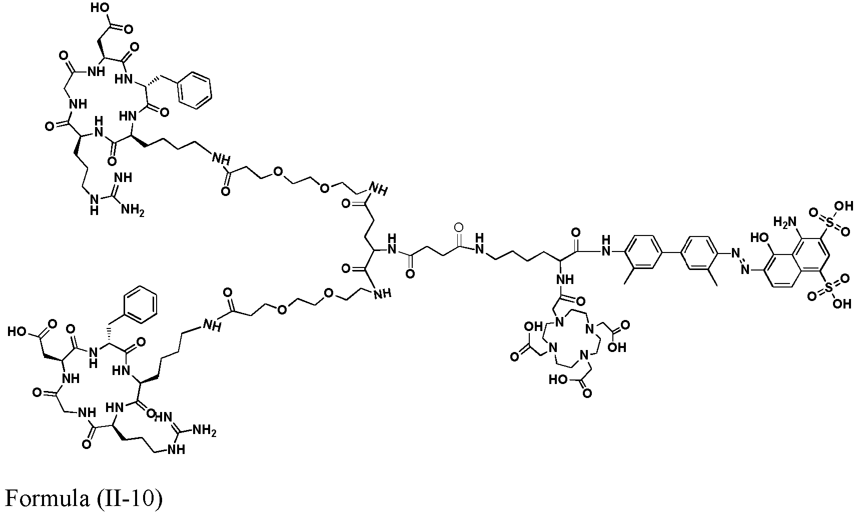

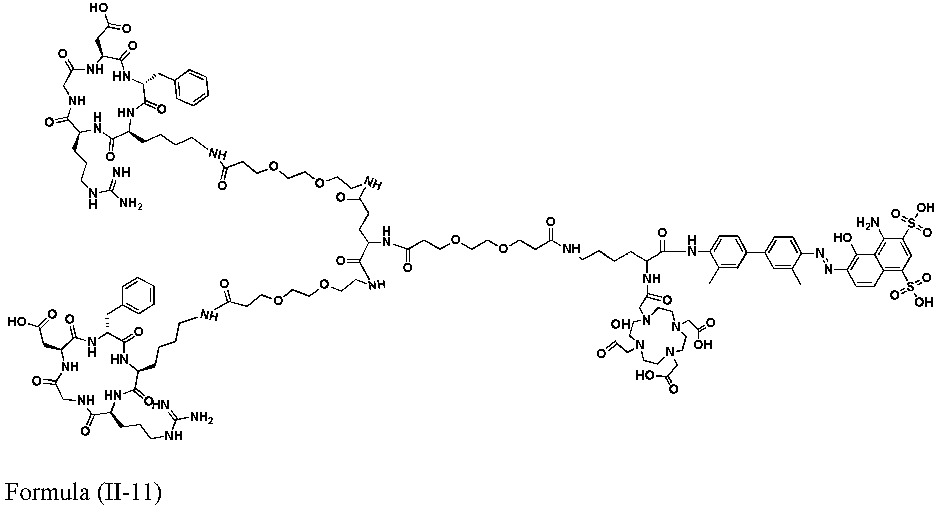

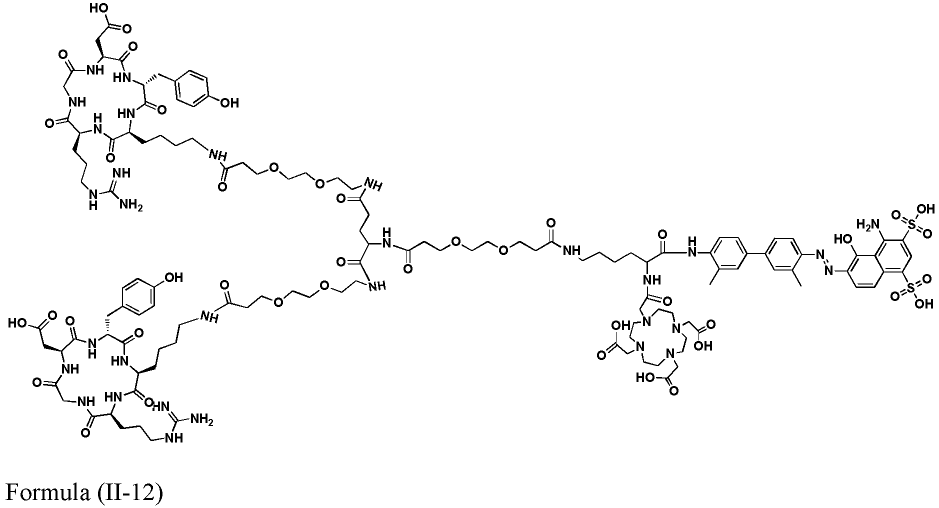

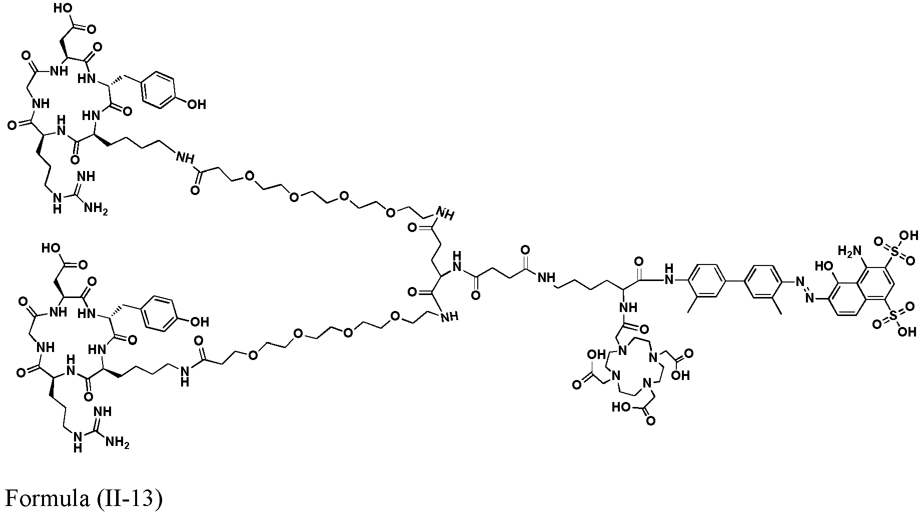

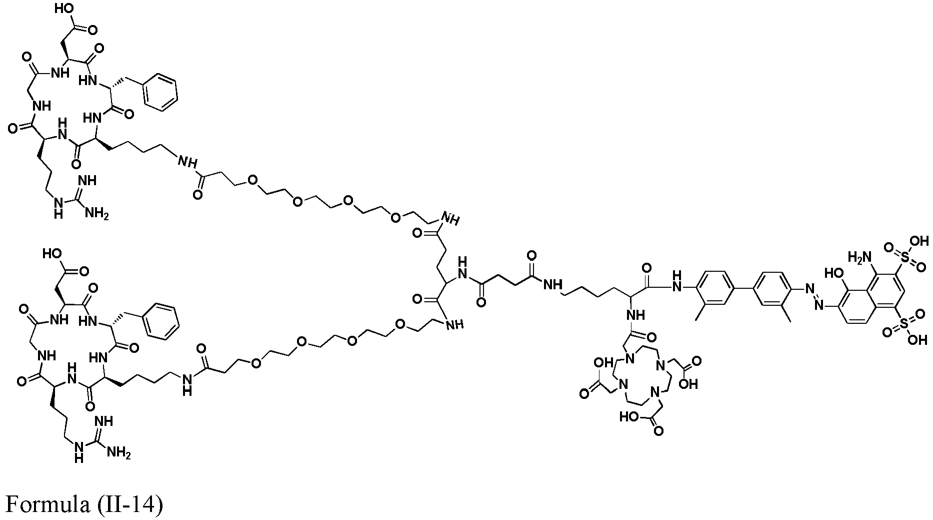

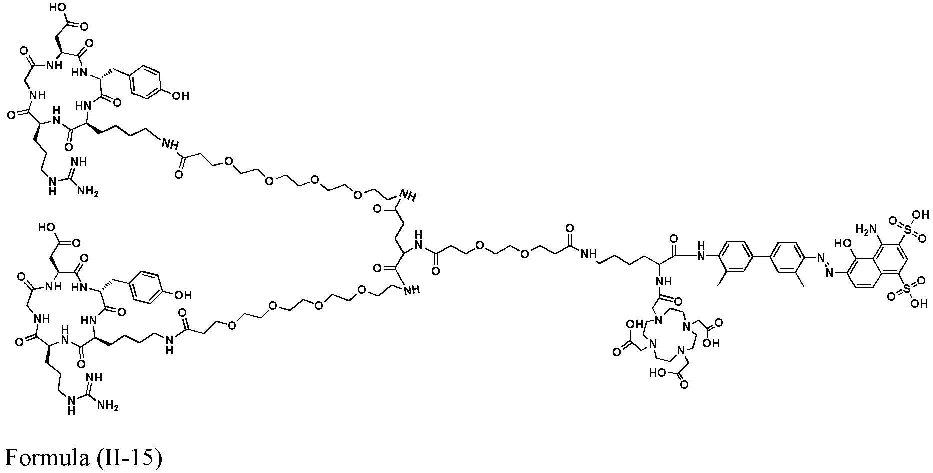

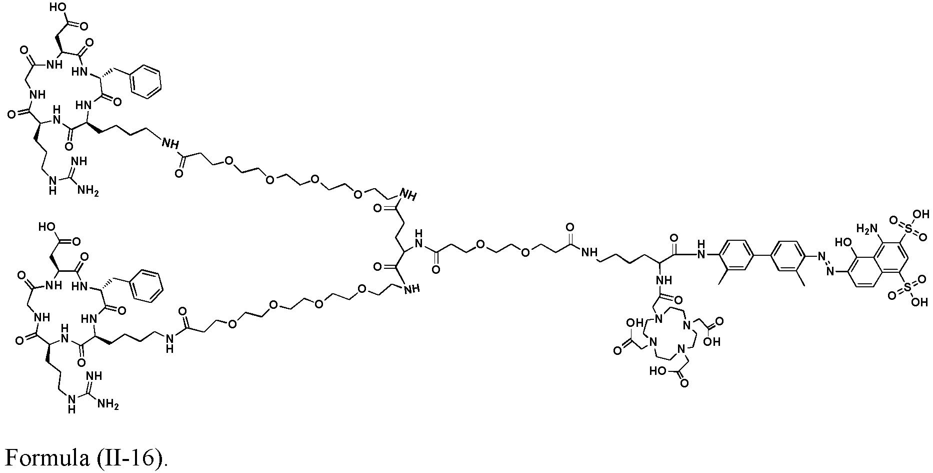

### Example 17: Preparation of a radionuclide ⁶⁸Ga labeled 2RGD-EB complex

Wet method: A hydrochloric acid solution of about 18.5-1,850 MBq of ⁶⁸GaCl₃ (rinsed with a germanium-gallium generator) was added into a centrifuge tube containing 0.5 mL of an acetic acid-acetate solution (1.0 g/L) of the compound H prepared in Example 1, and a reaction was carried out at 37°C for 20 min. A small C₁₈ separation column was taken, slowly rinsed with 10 mL of anhydrous ethanol first, and then rinsed with 10 mL of water. After being diluted with 10 mL of water, a labeled solution was loaded to the separation column, treated with 10 mL of water to remove unlabeled ⁶⁸Ga ions first, and then rinsed with 0.3 mL of a 10 mM ethanol solution of HCl to obtain a ⁶⁸Ga labeled 2RGD-EB complex. The rinsed solution was diluted with normal saline and then subjected to sterile filtration to obtain an injection of the ⁶⁸Ga labeled 2RGD-EB complex.

Freeze-drying method: A hydrochloric acid solution of about 18.5-1,850 MBq ⁶⁸GaCl₃ (rinsed with a germanium-gallium generator) was added into a freeze-dried medicine kit containing the compound H and mixed uniformly to carry out a reaction at 37°C for 20 min. A small C₁₈ separation column was taken, slowly rinsed with 10 mL of anhydrous ethanol first, and then rinsed with 10 mL of water. After being diluted with 10 mL of water, a labeled solution was loaded to the separation column, treated with 10 mL of water to remove unlabeled ⁶⁸Ga ions first, and then rinsed with 0.3 mL of a 10 mM ethanol solution of HCl to obtain a rinsed solution of a complex. The rinsed solution was diluted with normal saline and then subjected to sterile filtration to obtain an injection of the ⁶⁸Ga labeled 2RGD-EB complex.

### Analysis of experimental examples and application effect

### 1. Analysis and identification by HPLC

An HPLC system was as follows: SHIMADZULC-20A; and a C₁₈ chromatographic column (YMC, 3 µm, 4.6*150 mm) used for analysis. A detection wavelength was 254 nm, a flow rate was 1 mL/min, and a rinsing gradient was as follows: at 0-3 min, remaining 10% of acetonitrile and 90% of water (50 mM ammonium acetate) unchanged; at 3-16 min, increased to 90% of acetonitrile and 10% of water (50 mM ammonium acetate); at 16-18 min, remaining 90% of acetonitrile and 10% of water (50 mM ammonium acetate); at 18-20 min, reduced to 10% of acetonitrile and 90% of water (50 mM ammonium acetate); and at 20-22 min, remaining 10% of acetonitrile and 90% of water (50 mM ammonium acetate). A labeling system of the 2RGD-EB (compound H) prepared in Example 1 is shown in FIG. 2.

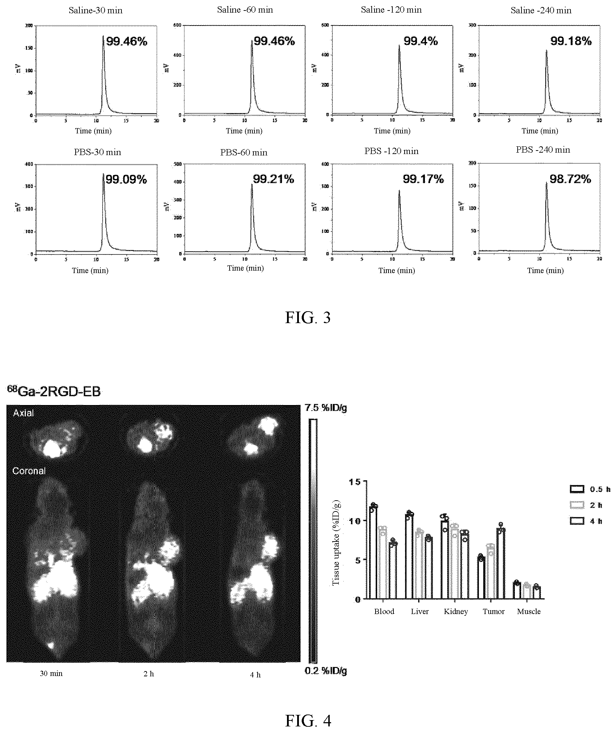

20 µL of a solution of the ⁶⁸Ga-2RGD-EB (with an activity of 3.7 MBq/20 µL) prepared in Example 17 was sucked and added into a centrifuge tube containing 100 µL of normal saline or PBS (with a pH value of 7.4) for co-incubation at 37°C for 0.5 h, 1 h, 2 h, and 4 h to obtain a co-incubation solution. 20 µL of the co-incubation solution was taken, filtered with a 0.22 µm needle type filter membrane, and analyzed by HPLC to obtain a radiochemical purity. Test results are shown in FIG. 3. The ⁶⁸Ga-2RGD-EB is not obviously decomposed after incubation in the PBS and the normal saline and has a radiochemical purity of greater than 98%, indicating that the ⁶⁸Ga-2RGD-EB prepared by the present invention has excellent stability.

### 2. MicroPET imaging of a ⁶⁸Ga labeled 2RGD-EB complex in U87 tumor-bearing mice

⁶⁸Ga-2RGD-EB was prepared by a method in Example 17. U87 tumor-bearing mice were administered with 7.4 MBq of the ⁶⁸Ga-2RGD-EB by tail intravenous injection, and then subjected to MicroPET imaging after administration for 30 min, 120 min, and 240 min under anesthetization with isoflurane, respectively. Results are shown in FIG. 4. In FIG. 4, a right side shows, from left to right, drug uptake situations of different tissues or organs of the mice at different time points after the injection in five groups of blood, liver, kidney, tumor and muscle, where the corresponding time, from left to right, in each group is 0.5 h, 2 h, and 4 h. The results show that at the test time points, tumor uptake is increased over time.

In summary, a 2RGD-EB structure is developed in the present invention, which can improve the tumor uptake and the retention time and is expected to be used in diagnosis or treatment of diseases characterized by over-expression of an integrin αᵥβ₃.

Although the present invention is described in detail by general description, specific embodiments and tests herein, it is obvious to persons skilled in the art that some modifications or improvements can be made on the basis of the present invention. Therefore, the modifications or improvements made without deviating from the spirit of the present invention fall within the scope of protection required by the present invention.

## Claims

1. An RGD dimer compound, having a structure shown in Formula (I) or Formula (I-1), 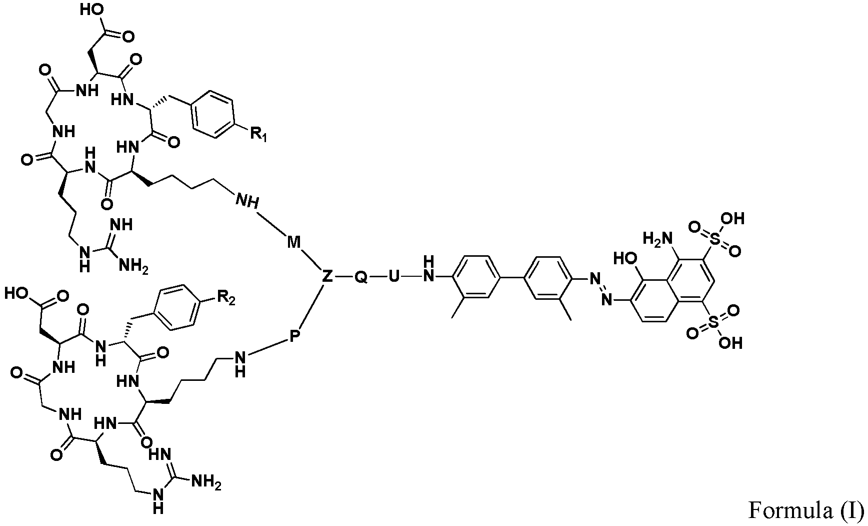 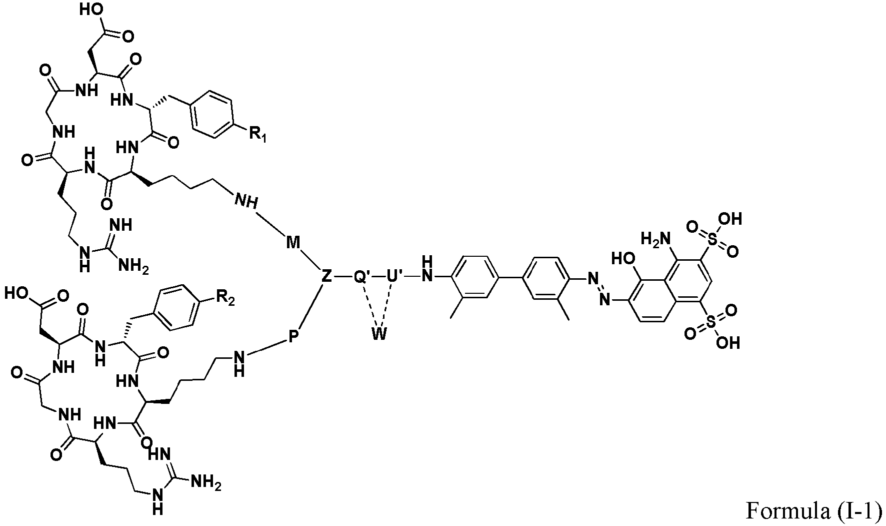 wherein:
R₁ and R₂ are independently selected from OH or H;
M and P are the same or different, and are independently selected from 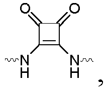 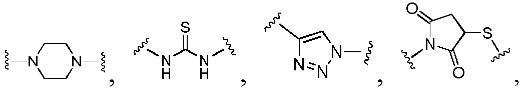 or -(CH₂)ₙ-; when the M and the P are -(CH₂)ₙ-, n is an integer ranging from 0 to 30, each -CH₂- is individually substituted or unsubstituted with -O-, -NH-, -(CO)-, -NH(CO)-, or -(CO)-NH-, and substitution occurs when no two adjacent -CH₂- groups are substituted;
Z is selected from 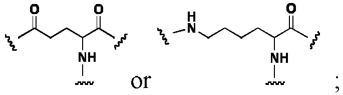
Q and U exist or do not exist, and are independently selected from 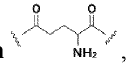 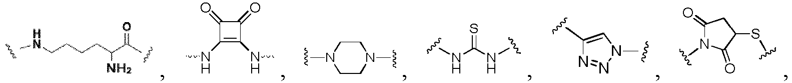 or -(CH₂)ₙ-; when the Q and the U are -(CH₂)ₙ-, n is an integer ranging from 0 to 30, each -CH₂- is individually substituted or unsubstituted with -O-, -NH-, -(CO)-, -NH(CO)-, or -(CO)-NH-, and substitution occurs when no two adjacent -CH₂- groups are substituted;
Q' and U' exist or do not exist, and any one structure in the Q' or the U' is connected with W; when the Q' or the U' exists and is connected with the W, the Q' and the U' are independently selected from 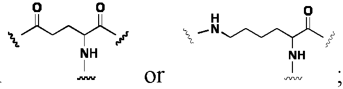 when the Q' or the U' exists and is not connected with the W, the Q' and the U' are independently selected from 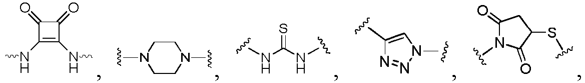 or -(CH₂)ₙ-; when the Q' and the U' are -(CH₂)ₙ-, n is an integer ranging from 0 to 30, each -CH₂- is individually substituted or unsubstituted with -O-, -NH-, -(CO)-, -NH(CO)-, or -(CO)-NH-, and substitution occurs when no two adjacent -CH₂- groups are substituted; and
the W is a group capable of being chelated with a radionuclide, and is any one structure selected from 1,4,7,10-tetraazacyclododecane-N,N',N,N'-tetraacetic acid (DOTA), ethylenediamine tetraacetic acid (EDTA), 1,4,7-triazacyclononane-1,4,7-triacetic acid (NOTA), triethylenetetramine (TETA), iminodiacetic acid, diethylenetriamine-N,N,N',N',N'-pentaacetic acid (DTPA), bis-(carboxymethylimidazole)glycinate, or 6-hydrazinonicotinic acid (HYNIC).

2. The RGD dimer compound according to claim 1, wherein the RGD dimer compound shown in Formula (I-1) has a structure shown in any one of Formula (II-1) to Formula (II-16):

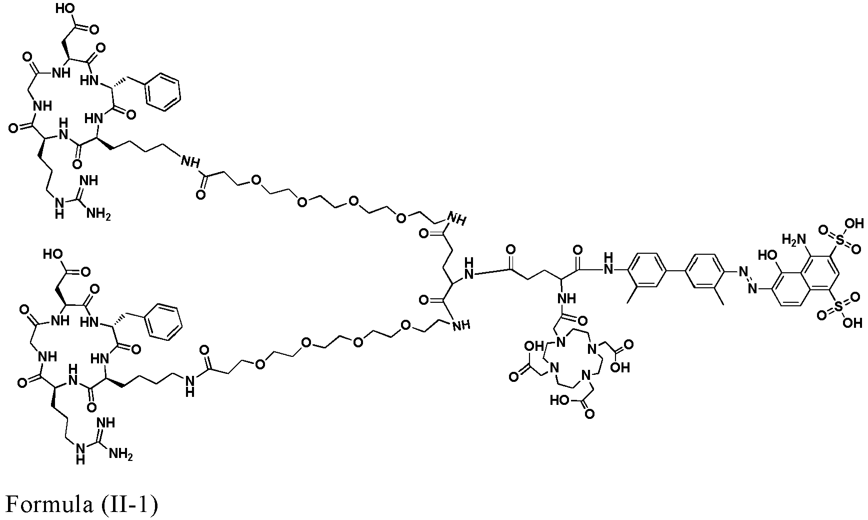

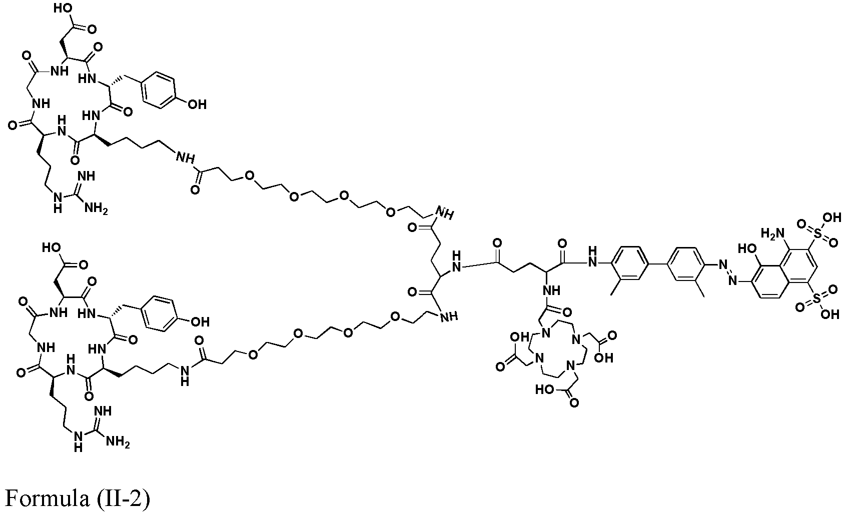

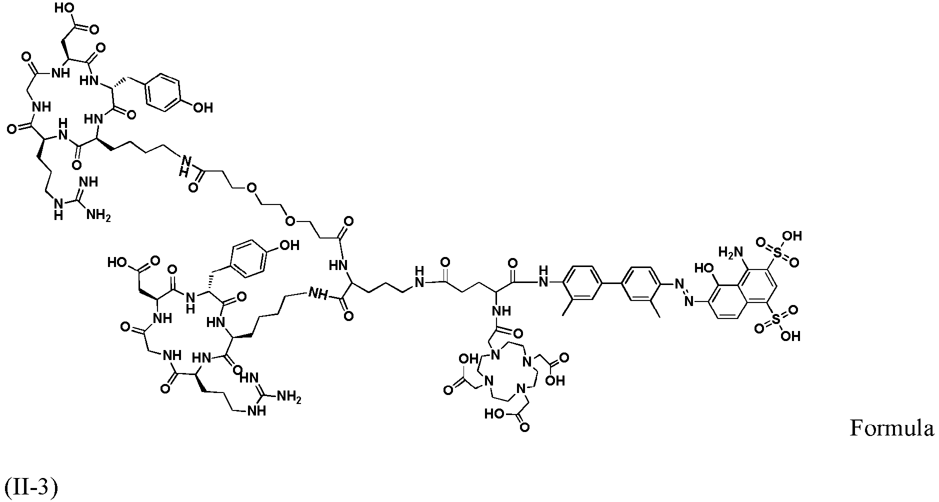

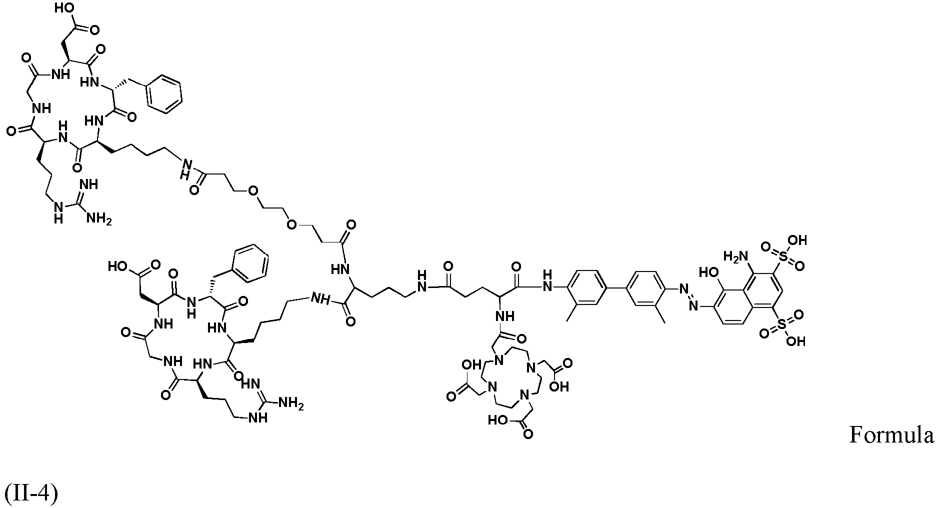

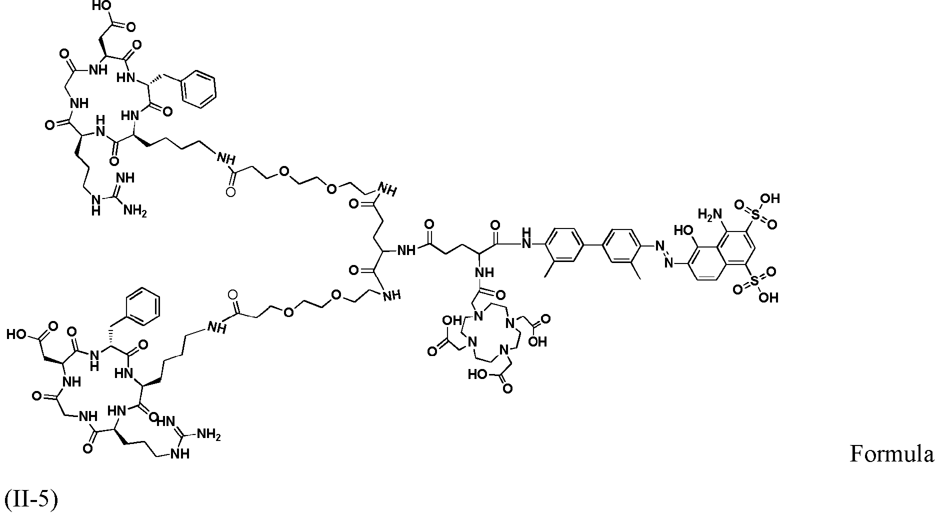

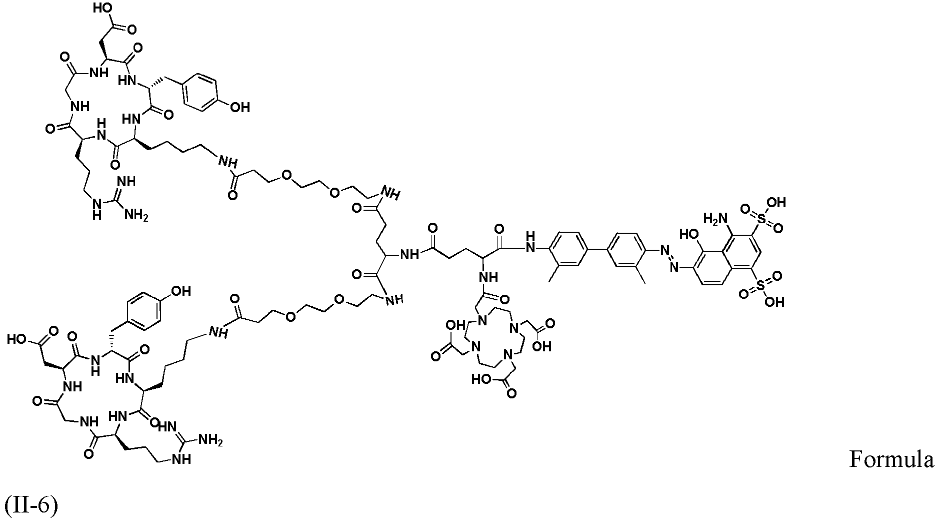

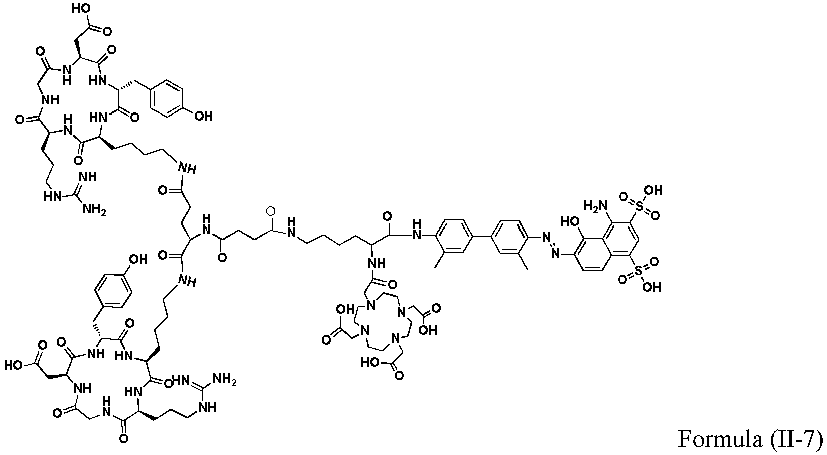

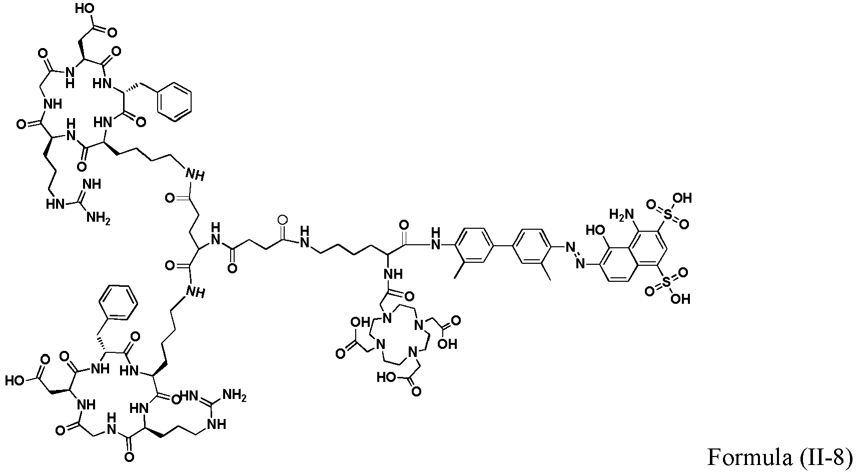

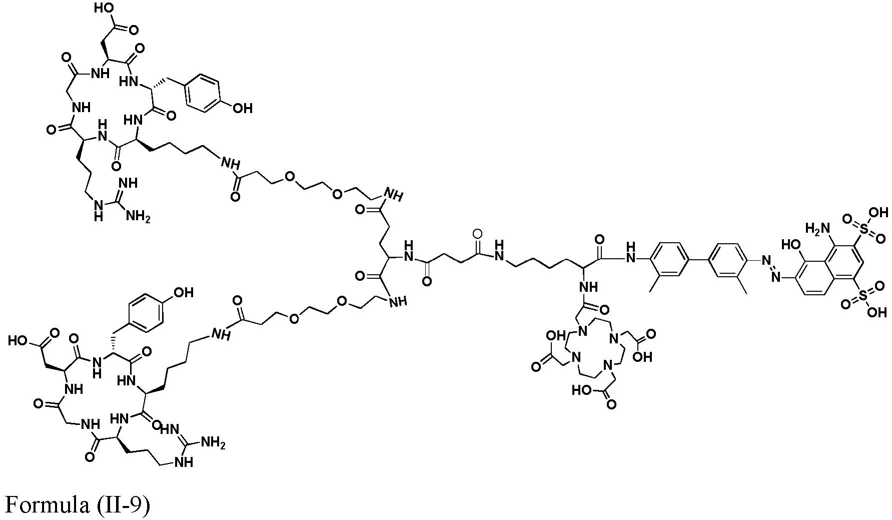

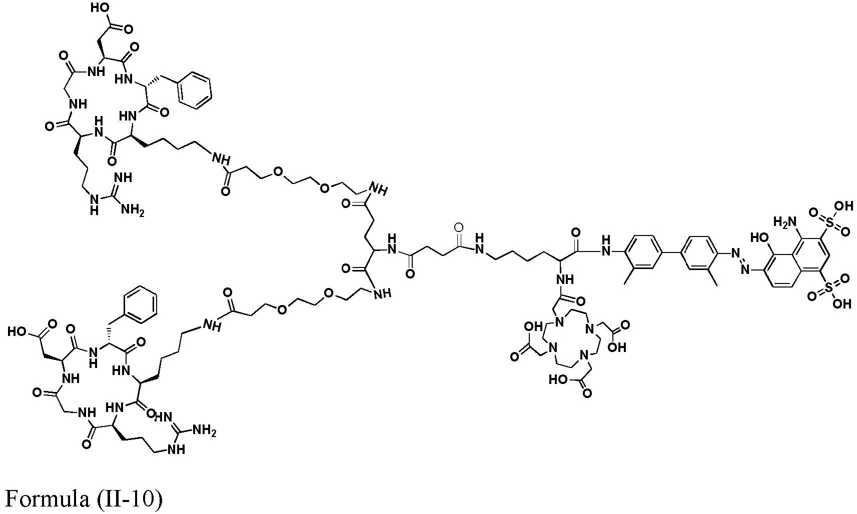

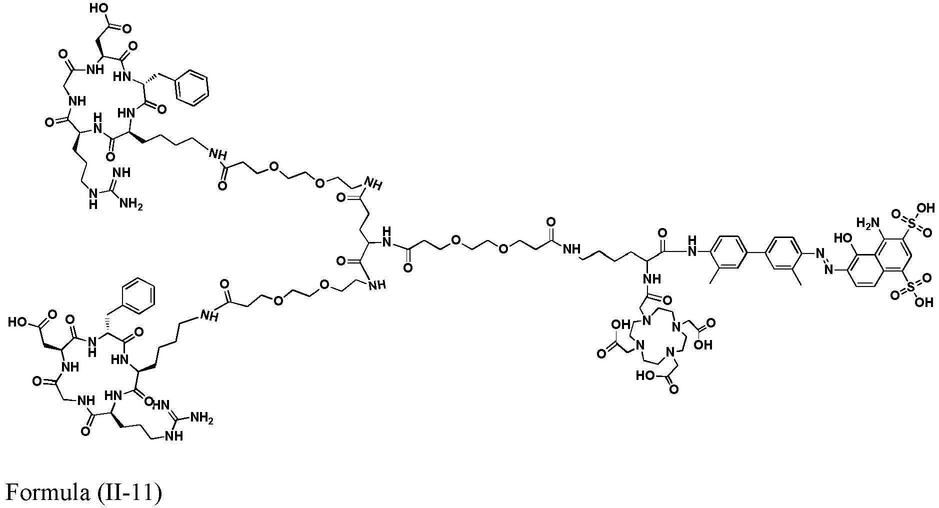

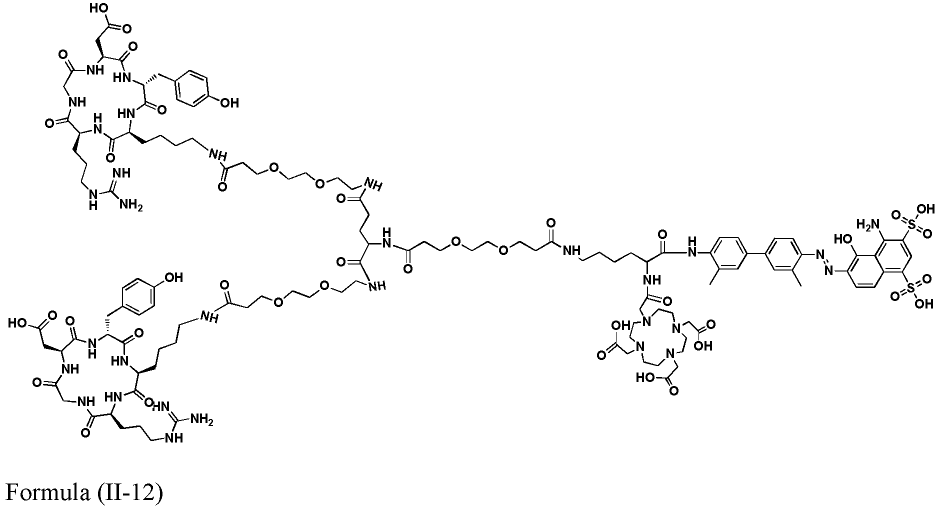

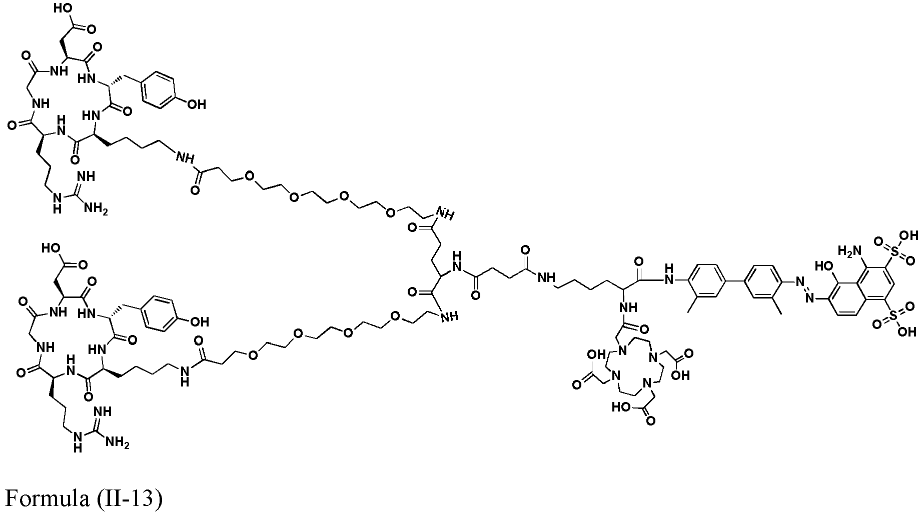

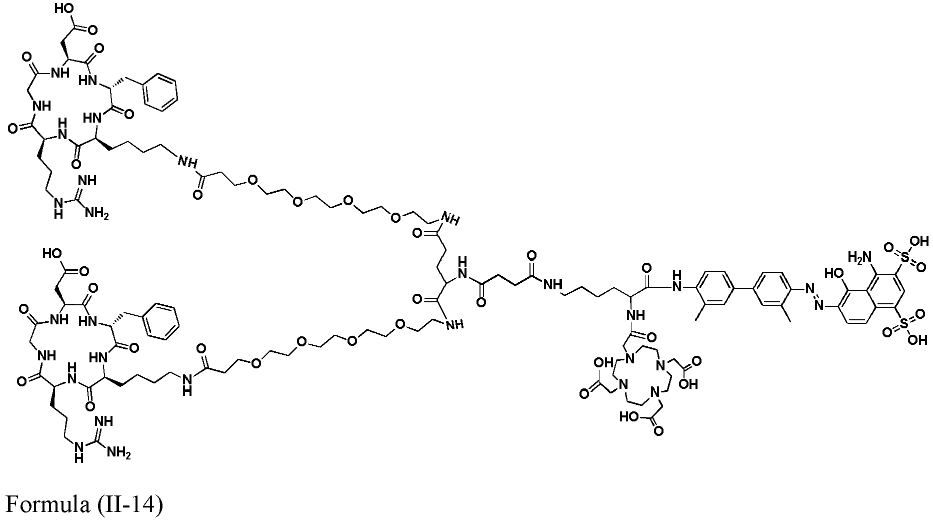

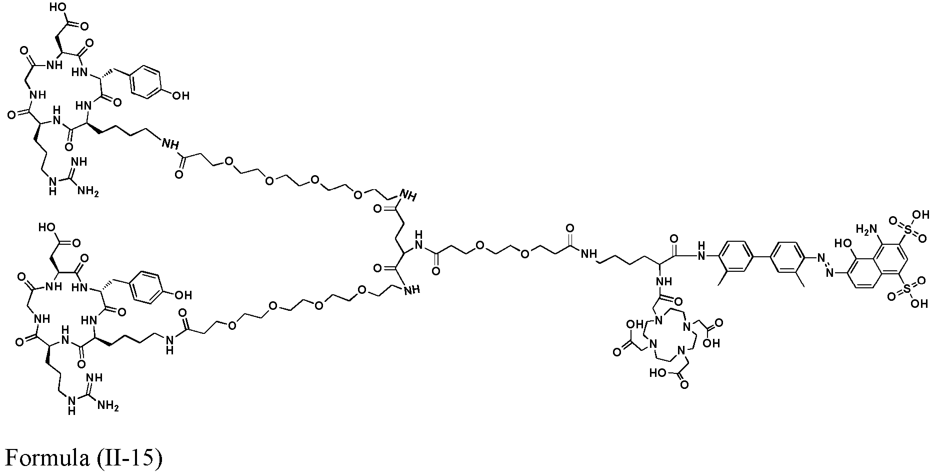

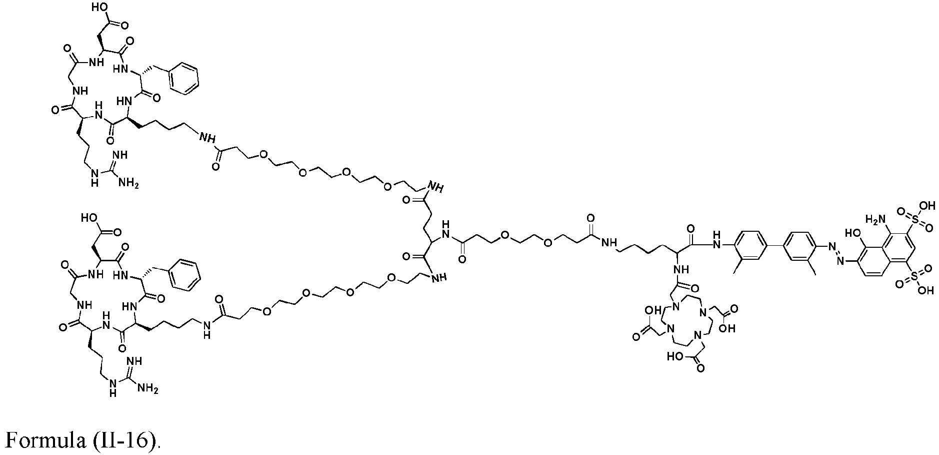

3. A radionuclide labeled compound, formed by chelating a radioisotope on a group W capable of being chelated with a radionuclide with the compound shown in Formula (I-1) according to any one of claims 1-2 as a ligand.

4. The radionuclide labeled compound according to claim 3, wherein the radioisotope is an isotope emitting α rays, an isotope emitting β rays, an isotope emitting γ rays, an isotope emitting Auger electrons, or an isotope emitting X rays.

5. The radionuclide labeled compound according to claim 3, wherein the radioisotope is any one of ¹⁸F, ⁵¹Cr, ⁶⁴Cu, ⁶⁷Cu, ⁶⁷Ga, ⁶⁸Ga, ⁸⁹Zr, ¹¹¹In, ^{99m}Tc, ¹⁸⁶Re, ¹⁸⁸Re, ¹³⁹La, ¹⁴⁰La, ¹⁷⁵Yb, ¹⁵³Sm, ¹⁶⁶Ho, ⁸⁶Y, ⁹⁰Y, ¹⁴⁹Pm, ¹⁶⁵Dy, ¹⁶⁹Er, ¹⁷⁷Lu, ⁴⁷Sc, ¹⁴²Pr, ¹⁵⁹Gd, ²¹²Bi, ²¹³Bi, ⁷²As, ⁷²Se, ⁹⁷Ru, ¹⁰⁹Pd, ¹⁰⁵Rh, ^{101m}Rh, ¹¹⁹Sb, ¹²⁸Ba, ¹²³I, ¹²⁴I, ¹³¹I, ¹⁹⁷Hg, ²¹¹At, ¹⁵¹Eu, ¹⁵³Eu, ¹⁶⁹Eu, ²⁰¹Tl, ²⁰³Pb, ²¹²Pb, ¹⁹⁸Au, ²²⁵Ac, ²²⁷Th, or ¹⁹⁹Ag.

6. The radionuclide labeled compound according to claim 3, wherein the radioisotope is ¹⁸F, ⁶⁴Cu, ⁶⁸Ga, ⁸⁹Zr, ⁹⁰Y, ¹¹¹In, ^{99m}Tc, ¹⁷⁷Lu, ¹⁸⁸Re, or ²²⁵Ac.

7. A pharmaceutical composition, comprising or composed of i) the RGD dimer compound according to any one of claims 1-2 or the radionuclide labeled compound according to claim 3, and ii) at least one pharmaceutically acceptable carrier and/or excipient.

8. Use of the RGD dimer compound according to any one of claims 1-2, the radionuclide labeled compound according to any one of claims 3-6, or the pharmaceutical composition according to claim 7 in preparation of drugs for diagnosis or treatment of diseases **characterized by** over-expression of an integrin αᵥβ₃ in animals or human objects.

9. The use according to claim 8, wherein the diseases **characterized by** over-expression of the integrin αᵥβ₃ comprise lung cancer, glioma, neuroglioma, breast cancer, pancreatic cancer, small intestine cancer, colon cancer, rectal cancer, head and neck cancer, ovarian cancer, hepatocellular carcinoma, esophageal cancer, hypopharyngeal carcinoma, nasopharyngeal carcinoma, laryngeal carcinoma, myeloma cells, bladder cancer, cholangiocellular carcinoma, clear cell renal cell carcinoma, neuroendocrine neoplasm, carcinogenic osteomalacia, sarcoma, cancer of unknown primary (CUP), thymic carcinoma, astrocytoma, cervical cancer, or prostatic cancer.

10. A kit, comprising or composed of the RGD dimer compound according to any one of claims 1-2, the radionuclide labeled compound according to any one of claims 3-6, or the pharmaceutical composition according to claim 7.
